(19) **Europäisches Patentamt / European Patent Office / Office européen des brevets**

(11) **EP 4 692 320 A1**

(12) **EUROPEAN PATENT APPLICATION**
published in accordance with Art. 153(4) EPC

(43) Date of publication:
**11.02.2026 Bulletin 2026/07**

(21) Application number: **24792770.0**

(22) Date of filing: **19.04.2024**

(51) International Patent Classification (IPC):
**C12N 5/071** (2010.01)  **C12Q 1/02** (2006.01)

(52) Cooperative Patent Classification (CPC):
**C12N 5/06; C12Q 1/02**

(86) International application number:
**PCT/JP2024/015630**

(87) International publication number:
**WO 2024/219499 (24.10.2024 Gazette 2024/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.04.2023 JP 2023069093**

(71) Applicants:
- **The University of Tokyo**
  **Bunkyo-ku, Tokyo 113-8654 (JP)**
- **Hilung Inc.**
  **Kyoto-shi, Kyoto 606-8304 (JP)**

(72) Inventors:
- **TAKEUCHI Shoji**
  **Tokyo 113-8654 (JP)**
- **MORIMOTO Yuya**
  **Tokyo 113-8654 (JP)**
- **TANI Yuta**
  **Tokyo 113-8654 (JP)**
- **SAWAYAMA Jun**
  **Tokyo 113-8654 (JP)**
- **IKEO Satoshi**
  **Tokyo 113-8654 (JP)**
- **YAMAMOTO Yuki**
  **Kyoto-shi, Kyoto 606-8304 (JP)**
- **NAGAMOTO Tetsuharu**
  **Kyoto-shi, Kyoto 606-8304 (JP)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

(54) **STRUCTURE AND EVALUATION METHOD USING STRUCTURE**

(57)    The present disclosure aims to provide a structure that allows access to the lumen of a cellular structure. The present disclosure provides a structure including: a sac-like cellular structure; and a tubular body, wherein the cellular structure includes a lumen and a cell layer at least partially covering the lumen, the cell layer is composed of cells, and the tubular body is configured to allow the lumen of the cellular structure to be in communication with an outside of the cellular structure.

FIG. 4A

FIG. 4B

EP 4 692 320 A1

## Description

TECHNICAL FIELD

The present disclosure relates to a structure and an evaluation method using the structure.

BACKGROUND ART

In order to mimic the functions of in-vivo organs or internal organs in an ex vivo environment, attempts have been made to induce organoids and the like. Methods for inducing various organoids such as alveolar organoids and intestinal organoids using induced pluripotent stem cells (iPS cells) or embryonic stem cells have already been established (Non-Patent Literature 1).

Citation List

Non-Patent Literature

Non-Patent Literature 1: Yamamoto Y et al., Nat Methods. 2017 Nov; 14(11): 1097-1106.

SUMMARY OF INVENTION

Technical Problem

Evaluation of the functionality of a formed organoid is generally performed by applying a stimulus to the organoid via a culture medium. However, it has not yet become possible to evaluate the functionality of the organoid by applying a stimulus to the organoid from within its lumen.

In light of the above circumstances, it is a primary object of the present disclosure to provide a structure that allows access to the lumen of a cellular structure such as, for example, an organoid. It is a secondary object of the present disclosure to provide a novel pulmonary acinus organoid capable of being used as the above-described organoid.

Solution to Problem

In order to achieve the above objects, the present disclosure provide a structure including:

    a sac-like cellular structure; and
    a tubular body,
    wherein the cellular structure includes a lumen and a cell layer at least partially covering the lumen,
    the cell layer is composed of cells, and
    the tubular body is configured to allow the lumen of the cellular structure to be in communication with an outside of the cellular structure.

The present disclosure also provides an evaluation method using the structure of the present disclosure, including the steps of:

    preparing a coexistence system in which a test substance and the cellular structure are present together; and
    evaluating the cellular structure and/or the test substance present in the coexistence system.

The present disclosure also provides a pulmonary acinus organoid including two or more sac-like cellular structures,

    wherein each of the cellular structures includes a lumen and two cell layers at least partially covering the lumen,
    each of the two cell layers includes alveolar epithelial cells, fibroblasts, and/or vascular endothelial cells, and
    the lumen of one of the cellular structures is in communication with the lumen of the at least one other cellular structure.

The present disclosure also provides a method for producing a pulmonary acinus organoid, including the step of culturing two or more spheroids in the presence of a differentiation-inducing factor for inducing differentiation into pulmonary acinus organoids, wherein the spheroids are each composed of lung progenitor cells, lung fibroblasts, and/or vascular endothelial cells.

Advantageous Effects of Invention

The present disclosure can provide a structure that allows access to the lumen of a cellular structure such as, for example, an organoid. The present disclosure also can provide a novel pulmonary acinus organoid capable of being used as the above-described organoid.

BRIEF DESCRIPTION OF DRAWINGS

    [FIG. 1] FIG. 1 shows graphs showing the average value of the major and minor axes of spheroids and graphs showing the circularity of the spheroids in Example 1.
    [FIG. 2] FIG. 2 shows photographs of images of stained alveolar organoids in Example 1.
    [FIG. 3] FIG. 3 shows photographs of images of stained alveolar organoids in Example 1.
    [FIG. 4] FIG. 4 shows photographs illustrating a method for puncturing an organoid using a puncture device in Example 1.
    [FIG. 5] FIG. 5 is a graph showing the expansion ratio of an organoid in Example 1.
    [FIG. 6] FIG. 6 shows photographs showing the results of observing a constructed organoid in Example 2.

[FIG. 7] FIG. 7 shows photographs and a graph, showing the results obtained when expansion and contraction of alveolar-like structures constituting a pulmonary acinus-like organoid were caused using the puncture device in Example 2.

[FIG. 8] FIG. 8 shows photographs showing the results of adding various drugs to pulmonary acinus-like organoids in Example 2.

[FIG. 9] FIG. 9 shows photographs and a graph, showing the results obtained when expansion and contraction of an alveolar organoid treated by adding bleomycin were caused using the puncture device in Example 2.

DESCRIPTION OF EMBODIMENTS

<Definition>

As used herein, the term "organoid" refers to an artificially induced organ and/or artificially induced internal organ designed to mimic a biological organ and/or biological internal organ. Typically, organoids are named according to organs or internal organs they mimic.

In the present disclosure, the term "spheroid" refers to a cell aggregate. Spheroids may be spherical in shape, for example.

As used herein, the term "cell polarity" refers to asymmetry within a cell. In the case of epithelial cells, their cell polarity may be, for example, apical-basolateral cell polarity. The cell polarity can be evaluated, for example, based on the localization of molecules present in a cell. When the cell is an epithelial cell and exhibits apical-basolateral cell polarity, the apical side can also be referred to as, for example, the side on which the Zonula occludens (ZO)-1 gene and/or Crumbs (CRB)-3 gene is expressed, and the basolateral side can also be referred to as the side on which neither the ZO-1 gene nor CRB-3 gene is expressed.

As used herein, the term "lung progenitor cells" refers to cells that are destined to differentiate into alveolar epithelial cells and/or airway epithelial cells upon embryologically appropriate stimulation. The lung progenitor cells express genes such as carboxypeptidase M (CPM), NK2 homeobox 1 (NKX2.1 or NKX2-1), SRY-box 9 (SRY (sex determining region Y)-box 9, SOX9), SRY-box 2 (SRY (sex determining region Y)-box 2, SOX2), and/or forkhead box protein 2A (FOXA2). The lung progenitor cells are preferably CPM-positive and/or NKX2.1-positive cells.

In the present disclosure, the term "epithelial cells" refers to cells that constitute the body surface or the surface of luminal organs.

As used herein, the term "alveolar epithelial cells" refers to epithelial cells present in the alveoli of the lungs. The alveolar epithelial cells may be, for example, alveolar epithelial type I cells and/or alveolar epithelial type II cells.

As used herein, the term "alveolar epithelial type I cells" refers to epithelial cells that are histologically flat in shape and express PDPN (Podoplanin), AGER (Advanced Glycosylation End-Product Specific Receptor), CAV1 (Caveolin 1), HOPX (HOP Homeobox), and/or AQP5 (Aquaporin 5).

As used herein, the term "alveolar epithelial type II cells" refers to epithelial cells that produce pulmonary surfactant proteins such as SFTPC (Surfactant protein C) and SFTPB (Surfactant protein B) and express SFTPA (Surfactant protein A), SFTPC (Surfactant protein C), SFTPB (Surfactant protein B), SFTPD (Surfactant protein D), EpCAM (Epithelial cell adhesion molecule), LPCAT1 (lysophosphatidylcholine acyltransferase 1), ABCA3 (ATP-binding cassette sub-family A member 3), DCLAMP (lysosome-associated membrane glycoprotein 3), and/or SLC34A2 (sodium-dependent phosphate transport protein 2B).

As used herein, the term "airway epithelial progenitor cells" refers to cells that are destined to differentiate into ciliated airway epithelial cells, CFTR-positive airway epithelial cells, mucus-producing airway cells, airway basal epithelial cells, neuroendocrine epithelial cells, and/or club cells upon embryologically appropriate stimuli and express FOXJ1 (Forkhead box protein J1), CFTR (Cystic fibrosis transmembrane conductance regulator), P63 (transformation-related protein 63, TP63), MUC5AC (Mucin 5AC), and/or NKX2.1.

As used herein, the term "airway epithelial cells" refers to epithelial cells present in the central and peripheral airways of the lungs. Examples of the airway epithelial cells include ciliated airway epithelial cells, club cells, airway basal epithelial cells, mucus-producing airway cells, CFTR-positive epithelial cells, and neuroendocrine cells.

As used herein, the term "proximal airway epithelial progenitor cells" refers to cells that are ciliated airway epithelial cells, mucus-producing airway cells, airway basal epithelial cells, neuroendocrine epithelial cells, and/or club cells and express SOX2 and NKX2.1.

As used herein, the term "ciliated airway epithelial cells" refers to epithelial cells that are histologically defined as having numerous dynamic cilia per cell and morphologically classified into a "9 + 2" structure, and they express Sentan (SNTN), acetylated tubulin, FOXJ1, DNAH5 (dynein axonemal heavy chain 5), and/or NKX2.1.

As used herein, the term "club cells" refers to epithelial cells that produce cell-specific proteins such as SCGB1A1 (secretoglobin family 1A member 1) and SCGB3A2 (secretoglobin family 3A member 2), as with club cells abundant in the peripheral airways of the lungs.

As used herein, the term "fibroblasts" refer to one of various types of cells that constitute connective tissue. The fibroblasts produce proteins such as collagen, hyaluronic acid, and elastin. The fibroblasts are cells that express, for example, vimentin, ACTA2, COL1A1 (Collagen type I alpha 1 chain), ELN (Elastin), FN (Fibronectin), PDGFRA (Platelet-derived growth factor receptor alpha), POSTN (Periostin), and/or TAGLN (Transgelin).

As used herein, the term "lung fibroblasts" refers to fibroblasts isolated from the lungs.

As used herein, the term "vascular endothelial cells" refers to flat cells that constitute the surface of the intravascular lumen. The vascular endothelial cells are cells that express, for example, CD31, TIE2, CD144, CD41, VEGFR-1, VEGFR-2, and/or VEGFR-3.

As used herein, the term "pulmonary acinus" refers to a still smaller unit within a pulmonary lobule, which is the smallest part of the bronchi, and the pulmonary acinus is composed of respiratory bronchioles and alveoli.

As used herein, the term "positive (+)" means that, in an analysis method such as flow cytometry that performs detection utilizing an antigen-antibody reaction, a higher signal is detected as compared with negative control cells that do not express the antigen or with a negative control reaction using an antibody that does not react with the antigen.

As used herein, the term "negative (-)" means that, in an analysis method such as flow cytometry that performs detection utilizing an antigen-antibody reaction, an equivalent or lower signal is detected as compared with negative control cells that do not express the antigen or with a negative control reaction using an antibody that does not react with the antigen.

As used herein, the term "isolated" refers to a state where a substance of interest has been identified and separated and/or a state where a substance of interest has been collected from natural components in which the substance originally occurs. The "isolated" state can be achieved, for example, by performing at least one purification step.

As used herein, the term "protein" or "peptide" refers to a polymer composed of unmodified amino acids (natural amino acids), modified amino acids, and/or artificial amino acids.

As used herein, the term "nucleic acid" or "polynucleotide" refers to a polymer composed of deoxyribonucleotides (DNA), a polymer composed of ribonucleotides (RNA), and/or a polymer composed of modified nucleotides. The nucleic acid may be single-stranded or double-stranded.

The sequence information of proteins described herein or nucleic acids (e.g., DNA and RNA) encoding them is available from the Protein Data Bank, UniProt, GenBank, or the like.

The present disclosure will be described below with reference to illustrative examples. It is to be noted, however, that the present disclosure is not limited to the following examples etc. and any changes and modifications may be made therein. In the present disclosure, descriptions regarding one aspect or embodiment can also be applied to another aspect or embodiment, and vice versa, unless otherwise stated. In the present specification, when a numerical range is delimited with the use of "to", numerical values or values representing physical quantities indicated before and after "to" are also included in this numerical range. The expression "A and/or B" as used herein encompasses the cases of "only A", "only B", and "both A and B". In the following description, "mol/l" may also be indicated as "M".

<Structure>

Viewed from one aspect, the present disclosure provides a structure that allows access to the lumen of a cellular structure. The structure of the present disclosure includes a sac-like cellular structure and a tubular body. The cellular structure includes a lumen and a cell layer at least partially covering the lumen, the cell layer is composed of cells, and the tubular body is configured to allow the lumen of the cellular structure to be in communication with an outside of the cellular structure. Since the structure of the present disclosure is configured such that the tubular body allows communication between the inside and the outside of the cellular structure, the lumen of the cells is accessible via the tubular body. Therefore, according to the structure of the present disclosure, the effect of a test substance on the cellular structure can be evaluated, for example, by introducing the test substance into the lumen of the cellular structure via the tubular body.

The cellular structure is a sac-like, i.e., hollow, structure composed of cells. The "cellular structure" can also be referred to as, for example, a cell cluster or spheroid with a hollow space inside.

The lumen of the cellular structure may or may not be in communication with the outside of the cellular structure except for the region where it communicates with the outside via the tubular body to be described below. However, in the cellular structure, it is preferable that the lumen is not in communication with the outside of the cellular structure except for this region because, for example, it allows the cellular structure to be expanded/contracted by pressurizing/depressurizing the lumen.

The cellular structure includes, for example, a lumen (portion) and a cell layer at least partially covering the lumen. The lumen can also be referred to as, for example, a region at least partially surrounded by the cell layer.

The lumen may be, for example, a hollow space or a space partially or entirely occupied by any other component. In other words, the lumen may contain contents. The lumen may contain, as its contents, substances derived from the cells that constitute the cell layer, for example. When the lumen contains the cell-derived substances, the cell-derived substances occupy the lumen either partially or entirely. The cell-derived substances can be determined depending on the cells that constitute the cell layer, for example. Examples of the cell-derived substances include sugars, peptides, proteins or glycoproteins, and lipids. Examples of the sugars include hyaluronic acid and proteoglycan. Examples of the proteins include: mucins such as MUC1 (Mucin 1), MUC5 (Mucin 5), and MUC5AC (Mucin 5AC); collagens; pulmonary surfactant proteins such as SFTPA, SFTPB, SFTPC, and SFTPD; Clara cell secretory proteins (CCSPs); and SCGB3A2 (Secretoglobin 3A2). Examples of the lipids include phosphatidyl choline. When the cellular structure contains, for example, a sugar as its content, the occurrence of expansion or contraction due to an osmotic pressure difference can be suppressed at the time of inserting the tubular body toward the lumen from the outside of the cell structure. When the cell layer includes alveolar epithelial type II cells, examples of the contents include surfactant proteins and MUC1. When the cell layer includes goblet cells, examples of the contents include MUC5 and MUC5AC. When the cell layer includes club cells, examples of the contents include CCSP and SCGB3A2.

The cell layer is a layer composed of cells. The cell layer includes one or more types of cells. The number of cells included in the cell layer can be set as appropriate depending on, for example, the size of the cellular structure, and specifically may be, for example, $1 \times 10^1$ to $1 \times 10^8$, $1 \times 10^1$ to $1 \times 10^3$, $1 \times 10^2$ to $1 \times 10^7$, or $1 \times 10^3$ to $1 \times 10^7$.

The cell layer is composed of a single cell layer or a plurality of cell layers. Preferably, the cell layer is composed of a single cell layer because, for example, the cell layer exhibits excellent elasticity during pressurization/depressurization of the lumen.

The cells forming the cell layer are not limited to particular types of cells, and any cells may be selected depending on, for example, an organ or internal organ that the cellular structure mimics. The cells are preferably epithelial cells because, for example, they are highly elastic and allow the cellular structure to be expanded and contracted by pressurizing/depressurizing the lumen. Examples of the epithelial cells include: alveolar epithelial cells such as alveolar epithelial type I cells and alveolar epithelial type II cells; airway epithelial cells such as ciliated airway epithelial cells, club cells, airway basal epithelial cells, mucus-producing airway cells, CFTR-positive epithelial cells, and neuroendocrine cells; gastric epithelial cells such as surface mucous cells, glandular cells, and endocrine cells; small intestinal epithelial cells such as absorptive epithelial cells, goblet cells, Paneth cells, tuft cells, endocrine cells, M cells, and LGR5-positive stem cells; and large intestinal epithelial cells such as absorptive epithelial cells, goblet cells, and endocrine cells.

For example, an organoid may be used as the cellular structure. The organoid can be selected as appropriate depending on, for example, an organ or internal organ that the cellular structure mimics. The organoid may be, for example, a pulmonary acinus organoid, an alveolar organoid, an airway organoid, an intestinal organoid, a small intestinal organoid, a brain organoid, a cortical organoid, a retinal organoid, a cystic organoid, a gastric organoid, a kidney organoid, a bile duct organoid, an esophageal organoid, or a large intestinal organoid. The above-described respective organoids can be prepared according to the following Reference Literatures. The pulmonary acinus organoid can be prepared according to a method for producing a pulmonary acinus organoid to be described below. When the cellular structure is an organoid, the organoid is preferably derived from an organ or tissue having a tubular structure, and specific examples thereof include pulmonary acinus organoids, alveolar organoids, airway organoids, intestinal organoids, small intestinal organoids, brain organoids, cystic organoids, gastric organoids, bile duct organoids, esophageal organoids, and large intestinal organoids. When the cellular structure is an organoid, the cellular structure is preferably an organoid derived from an organ or tissue having a tubular structure and more preferably is a pulmonary acinus organoid or alveolar organoid, in terms of excellent elasticity during pressurization/depressurization of the lumen.

Reference Literatures:

Alveolar organoids:
Yamamoto Y et al., Nat Methods. 2017 Nov;14(11):1097-1106. doi: 10.1038/nmeth.4448. Epub 2017 Oct 2.
Airway organoids:
Konishi S et al., Stem Cell Reports. 2016 Jan 12;6(1):18-25. doi:10.1016/j.stemcr.2015.11.010. Epub 2015 Dec 24.
Intestinal organoids:
Spence JR et al. Directed differentiation of human pluripotent stem cells into intestinal tissue in vitro. Nature. 470, 105-9 (2011).
Small intestinal organoids:

Spence JR et al., Nature. 2011 Feb 3;470(7332):105-9. doi: 10.1038/nature09691. Epub 2010 Dec 12; and
Watson CL et al. An in vivo model of human small intestine using pluripotent stem cells. Nat Med. 20, 1310-4 (2014).

Brain organoids:

Lancaster MA et al. Cerebral organoids model human brain development and microcephaly. Nature. 501, 373-9 (2013);
Camp JG et al. Human cerebral organoids recapitulate gene expression programs of fetal neocortex development. Proc Natl Acad Sci U S A. 112, 15672-7 (2015);
Mariani J et al. FOXG1-Dependent Dysregulation of GABA/Glutamate Neuron Differentiation in Autism Spectrum Disorders. Cell. 162, 375-390 (2015);

Cugola FR et al. The Brazilian Zika virus strain causes birth defects in experimental models. Nature. 534, 267-71 (2016);

Garcez PP et al. Zika virus impairs growth in human neurospheres and brain organoids. Science. 352, 816-8 (2016); and

Gabriel E et al. CPAP promotes timely cilium disassembly to maintain neural progenitor pool. EMBO J. 35, 803-19 (2016).

Cortical organoids:

Otani T et al. 2D and 3D Stem Cell Models of Primate Cortical Development Identify Species-Specific Differences in Progenitor Behavior Contributing to Brain Size. Cell Stem Cell. 18, 467-80 (2016).

Retinal organoids:

Tucker BA et al. Duplication of TBK1 Stimulates Autophagy in iPSC-derived Retinal Cells from a Patient with Normal Tension Glaucoma. J Stem Cell Res Ther. 3, 161 (2014).

Cystic organoids:

Sampaziotis F et al. Cholangiocytes derived from human induced pluripotent stem cells for disease modeling and drug validation. Nat Biotechnol. 33, 845-852 (2015); and

Ogawa M et al. Directed differentiation of cholangiocytes from human pluripotent stem cells. Nat Biotechnol. 33, 853-61 (2015).

Kidney organoids:

Takasato M et al. Kidney organoids from human iPS cells contain multiple lineages and model human nephrogenesis. Nature. 526, 564-8 (2015).

Gastric organoids:

McCracken KW., et al. Nature. 2014 Dec 18;516(7531):400-4. doi: 10.1038/nature13863. Epub 2014 Oct 29.

Large intestinal organoids:

Munera JO et al., Cell Stem Cell. 2017 Jul 6;21(1):51-64.e6. doi: 10.1016/j.stem.2017.05.020. Epub 2017 Jun 22.

When the cellular structure is an organoid, the lumen of the organoid may be, for example, a hollow space or a space partially or entirely occupied by any other component. In other words, the lumen may contain contents. The lumen may contain, as its contents, substances derived from the cells constituting the organoid, for example. When the lumen contains the organoid-derived substances, the organoid-derived substances occupy the lumen either partially or entirely. The organoid-derived substances can be determined depending on the cells constituting the organoid, for example. For the organoid-derived substances, reference can be made to the above descriptions regarding the cell-derived substances, for example. When the cellular structure includes an alveolar organoid, examples of the contents include pulmonary surfactant proteins, lipids, and MUC1 proteins. When the cellular structure includes an airway organoid, examples of the contents include MUC5AC, MUC5B, and CCSP.

The cellular structure may have apical polarity on its lumen side or on the outer side, for example.

It is preferable that the cellular structure has apical polarity on the lumen side, i.e., the cells forming the cell layer are oriented such that they have apical polarity on the lumen side, because, for example, in testing of a test substance such as a drug, the cellular structure can be used under conditions more closely resembling in-vivo contact environments.

It is preferable that the cellular structure has apical polarity on the outer side, i.e., the cells forming the cell layer are oriented such that they have apical polarity on the outer side, because, for example, in testing of a test substance such as a drug, the cellular structure can be used under conditions more closely resembling in vivo contact environments.

The apical polarity of the cellular structure can be identified, for example, by examining the genes expressed on the apical side. Specifically, when the cells constituting the cellular structure are alveolar epithelial type II cells, examples of the gene expressed on the apical side include the MUC1 gene and the SLC34A2 gene. When the cells constituting the cellular structure are alveolar epithelial type I cells, examples of the gene expressed on the apical side include the PDPN gene and the AGER gene. When the cells constituting the cellular structure are ciliated airway epithelial cells, examples of the gene expressed on the apical side include the SNTN gene.

The arrangement of cells in the cellular structure can be achieved, for example, either through the method for inducing the cellular structure or by changing the orientation of apical polarity in the induced cellular structure. Specifically, the apical polarity can be changed using an extracellular matrix according to the following Reference Literature, for example. Reference Literature: Co, J.Y., Margalef-Catala, M., Monack, D.M. et al. Controlling the polarity of human gastrointestinal organoids to investigate epithelial biology and infectious diseases. Nat Protoc 16, 5171-5192 (2021).

The diameter of the cellular structure is, for example, 1 to 2000 $\mu$m, 1 to 1500 $\mu$m, 1 to 1000 $\mu$m, 1 to 500 $\mu$m, 5 to 500 $\mu$m, or 10 to 150 $\mu$m. In the present specification, the diameter of the cellular structure refers to the average value of the major axis (long axis) and minor axis (short axis) of the cellular structure. The diameter of the cellular structure can be measured, for example, according to a method described in the item (5) of Example 1 to be described below.

The cellular structure may be able to be expanded and/or contracted by changing pressure applied to the lumen via the tubular body. Specifically, the cellular structure may be expanded when pressure is applied to the lumen via the tubular body and may be contracted when reduced pressure is applied to the lumen via the tubular body, for example. The treatment for applying pressure or reduced pressure to the lumen of the cellular structure can be performed, for example, with reference to the item (10) of Example 1 to be described below.

The tubular body need only be configured such that, for example, it allows the lumen of the cellular structure to be in communication with the outside when disposed in the cellular structure, for example. The tubular body may be, for example, a tube or a tube with a tapered surface. When the tubular body is a tube with a tapered surface, the tubular body can be easily disposed in the cellular structure. When the tubular body is a tube with a tapered surface, for example, part or the whole of the tapered surface of the tubular body is disposed in the cellular structure. The tube with a tapered surface may be, for example, an injection needle, a microneedle, or a microcapillary. The material of the tubular body is not limited to a particular material, and may be, for example, glass or plastic. The shape of the tubular body may be either straight or branched, for example. When the tubular body is branched, the tubular body may have two openings or three or more openings. The branched tubular body may be, for example, a fork-shaped tubular body.

The tubular body is configured such that it allows the lumen of the cellular structure to be in communication with the outside when disposed in the cellular structure. In the present disclosure, the tubular body may be such that, for example, one of the openings thereof is not open. Also, the tubular body may be such that a hollow space thereof is partially or fully filled. When the tubular body is partially or fully filled, the tubular body is filled with a gas, liquid, and/or solid.

The outer diameter, the inner diameter, and the length of the tubular body can be set, for example, depending on the size of the cellular structure. Specifically, the outer diameter and the inner diameter of the tubular body may be set, for example, so as to be smaller than the diameter of the cellular structure. The length of the tubular body may be set, for example, so as to be greater than the thickness of the cellular structure as measured from the lumen to the outer periphery of the cellular structure. The diameter of the tubular body, i.e., the outer diameter or the inner diameter of the tubular body, is, for example, 1 to 1000 $\mu$m, 5 to 500 $\mu$m, 10 to 500 $\mu$m, or 10 to 150 $\mu$m.

In the structure of the present disclosure, one or more tubular bodies may be disposed in the cellular structure. By providing a plurality of tubular bodies, the structure of the present disclosure allows different treatments to be performed with respect to the lumen of the cellular structure, for example.

The structure of the present disclosure can be produced, for example, by introducing the tubular body into the cellular structure, more specifically, by puncturing the cellular structure with the tubular body and inserting the tubular body from the outside toward the lumen of the cellular structure. Alternatively, the structure of the present disclosure may be produced, for example, by placing the tubular body and then forming the cellular structure so as to surround the tubular body.

In the structure of the present disclosure, the cellular structure can be prepared as appropriate, for example, depending on the type of cells constituting the cellular structure. When the cellular structure is an organoid, for example, reference can be made to the above descriptions relating to the method for inducing organoids. As a specific example, when the organoid is an alveolar organoid, the alveolar organoid can be induced from, for example, alveolar progenitor cells. For the method for inducing the alveolar organoid from the alveolar progenitor cells, reference can be made to the description thereon in U.S. Patent No. 11,299,712, for example.

The alveolar organoid can be induced, for example, by culturing, preferably in a three-dimensional environment, the alveolar progenitor cells in the presence of an alveolar epithelial cell-inducing factor (induction step). The induction step can be performed, for example, by culturing the lung progenitor cells in the presence of a medium containing the alveolar epithelial cell-inducing factor. The lung progenitor cells are preferably CPM-positive lung progenitor cells. The lung progenitor cells are, for example, isolated lung progenitor cells and preferably isolated CPM-positive lung progenitor cells.

The medium can be prepared using, as a basal medium, a medium used for culturing animal cells. Examples of the basal medium include IMDM medium, Medium 199, Eagle's Minimum Essential Medium (EMEM), $\alpha$MEM medium, Dulbecco's Modified Eagle's Medium (DMEM), Ham's F12 medium, RPMI 1640 medium, Fisher's medium, Neurobasal Medium (manufactured by Life Technologies), and media obtained by mixing them in any combination. The medium may contain serum or may be free of serum. The media may contain, for example, one or more serum replacements such as albumin, transferrin, Knockout Serum Replacement (KSR, a serum replacement for FBS during culture of ES cells), N2 supplement (manufactured by Invitrogen), B27 supplement (manufactured by Invitrogen), fatty acids, insulin, ITS premix, collagen precursors, trace elements, 2-mercaptoethanol, and 3'-thioglycerol. The medium may further contain, for example, one or more components such as lipids, amino acids, L-glutamine, Glutamax (manufactured by Invitrogen), non-essential amino acids, vitamins, growth factors, low molecular weight compounds, antibiotics, antioxidants, pyruvic acid, buffers, and inorganic salts. The medium is preferably Ham's F12 medium containing bovine serum albumin (BSA), HEPES, calcium chloride, ITS premix, B27 supplement, penicillin, and streptomycin.

The alveolar epithelial cell-inducing factor is a factor capable of inducing alveolar epithelial type I cells and/or alveolar epithelial type II cells. The alveolar epithelial cell-inducing factor may be, for example, a steroid, a cAMP derivative, a phosphodiesterase inhibitor, and/or KGF, and it is preferable to use the steroid, the cAMP derivative, the phosphodiesterase inhibitor, and the KGF.

The steroid refers to a compound having a steroid skeleton. The steroid may be, for example, a steroidal anti-inflammatory drug, and specific examples thereof include glucocorticoids and their synthetic derivatives. Examples of the steroid include hydrocortisone, hydrocortisone succinate, prednisolone, methylprednisolone, methylprednisolone succinate, triamcinolone, triamcinolone acetonide, dexamethasone, and betamethasone. Of these, dexamethasone is preferable.

The steroid concentration in the medium is not limited to a particular concentration, and is, for example, 1 nM to 1 $\mu$M, specifically 5 to 900 nM, 10 to 800 nM, 20 to 700 nM, 30 to 600 nM, 40 to 500 nM, 50 to 400 nM, 60 to 300 nM, 70 to 200 nM, 80 to 100 nM, and preferably about 50 nM.

The cAMP derivative refers to a compound in which cyclic AMP is modified with a substituent(s). Examples of the cAMP derivative include cyclic adenosine monophosphate (cAMP), 8-bromo cyclic adenosine monophosphate (8-Br-cAMP), 8-chloro cyclic adenosine monophosphate (8-Cl-cAMP), 8-(4-Chlorophenylthio) cyclic adenosine monophosphate (8-CPT-cAMP), and Dibutyryl cyclic adenosine monophosphate (DB-cAMP). Of these, 8-Br-cAMP is preferable.

The cAMP concentration in the medium is not limited to a particular concentration, and is, for example, 1 nM to 1 $\mu$M, specifically 5 to 900 nM, 10 to 800 nM, 20 to 700 nM, 30 to 600 nM, 40 to 500 nM, 50 to 400 nM, 60 to 300 nM, 70 to 200 nM, 80 to 100 nM, and preferably about 100 nM.

The phosphodiesterase inhibitor refers to a compound that increases the intracellular concentration of cAMP or cGMP by inhibiting phosphodiesterase (PDE). Examples of the phosphodiesterase inhibitor include 1,3-Dimethylxanthine, 6,7-Dimethoxy-1-(3,4-dimethoxybenzyl)isoquinoline, 4-{[3',4'-(Methylenedioxy)benzyl]amino}-6-methoxyquinazoline, 8-methoxymethyl-3-isobutyl-1-methylxanthine, and 3-isobutyl-1-methylxanthine (IBMX). Of these, IBMX is preferable.

The phosphodiesterase inhibitor concentration in the medium is not limited to a particular concentration, and is, for example, 1 nM to 1 $\mu$M, specifically 5 to 900 nM, 10 to 800 nM, 20 to 700 nM, 30 to 600 nM, 40 to 500 nM, 50 to 400 nM, 60 to 300 nM, 70 to 200 nM, 80 to 100 nM, and preferably about 100 nM.

The KGF (Keratinocyte Growth Factor) or FGF7 (Fibroblast Growth Factor) is a protein known as a growth factor for keratinocytes. Human-derived KGF is, for example, a protein encoded by the polynucleotide identified by NCBI Accession Number NM_002009. The KGF may be, for example, in a state of being activated by cleavage with a protease. The activated KGF is available from Wako, for example.

The KGF concentration in the medium is not limited to a particular concentration, and is, for example, 10 ng/ml to 1 $\mu$g/ml, 20 to 900 ng/ml, 30 to 800 ng/ml, 40 to 700 ng/ml, 50 to 600 ng/ml, 60 to 500 ng/ml, 70 to 400 ng/ml, 80 to 300 ng/ml, or 90 to 200 ng/mll, and preferably about 10 ng/ml.

In the induction step, for example, a ROCK inhibitor may be added as the alveolar epithelial cell-inducing factor. The ROCK inhibitor is a compound capable of suppressing the function of Rho-kinase (ROCK). Examples of the ROCK inhibitor include Y-27632 ((+)-(R)-trans-4-(1-aminoethyl)-N-(4-pyridyl)cyclohexanecarboxamide dihydrochloride), Fasudil/HA1077 (5-(1,4-Diazepane-1-sulfonyl)isoquinoline), H-1152 ((S)-(+)-2-Methyl-1-[(4-methyl-5-isoquinolinyl)sulfonyl]homopiperazine), Wf-536 ((+)-(R)-4-(1-Aminoethyl)-N-(4-pyridyl) benzamide), and nucleic acid molecules (such as siRNA, shRNA, and an antisense) that suppress the expression of the ROCK protein. Of these, Y-27632 is preferable.

The ROCK inhibitor concentration in the medium is, for example, 1 nmol/l to 50 $\mu$mol/l, 10 nmol/l to 40 $\mu$mol/l, 50 nmol/l to 30 $\mu$mol/l, 100 nmol/l to 25 $\mu$mol/l, 500 nmol/l to 20 $\mu$mol/l, or 750 nmol/l to 15 $\mu$mol/l.

In the induction step, for example, three-dimensional culture of the alveolar epithelial progenitor cells is performed to promote maturation of the alveolar epithelial progenitor cells. The three-dimensional culture refers to culturing cells that are in the form of clusters (spheroids) in a suspended state. The three-dimensional culture can be performed, for example, using a cell culture insert, an extracellular matrix such as Matrigel, etc. available from BD.

The three-dimensional culture may be performed, for example, using only the lung progenitor cells or in the form of co-culture with other cells. Examples of other cells include human lung fibroblasts and human fetal lung fibroblasts. These other cells are available from, for example, American Type Culture Collection (ATCC) or DV Biologics. The mixing ratio between the alveolar epithelial progenitor cells (E) and other cells (O) (E:O (the number of cells) is, for example, 1:1 to 1000 or 1:10 to 500 and preferably around 1:50. The cell density in the medium is, for example, $0.5 \times 10^6$ to $1 \times 10^7$ cells/ml and preferably about $2.5 \times 10^6$ cells/ml.

When performing the three-dimensional culture, the above-described medium may be supplemented with an extracellular matrix. The quantitative ratio (M:E) between the medium (M) and the extracellular matrix (E) is, for example, 1:0.01 to 50 or 1:0.25 to 10 and preferably around 1:1.

The extracellular matrix is a supramolecular structure present outside cells. The extracellular matrix may be a naturally occurring extracellular matrix or an artificial extracellular matrix (such as a recombinant or a peptide hydrogel). The extracellular matrix may be, for example, a molecule such as collagen, proteoglycan, fibronectin, hyaluronic acid, tenascin, entactin, elastin, fibrillin, or laminin, or a fragment thereof. One type of extracellular matrix may be used, or two or more types of extracellular matrices may be used in combination. The extracellular matrix may be, for example, a preparation derived from cells, such as Corning Matrigel™. Examples of the artificial extracellular matrix include fragments of laminin and Corning PuraMatrix™.

The culture conditions used in the induction step are not limited to particular conditions, and may be set, for example, based on the culture conditions for the lung progenitor cells. Specifically, the culture temperature is, for example, about 30°C to 40°C and preferably about 37°C. The culture is performed, for example, in a $CO_2$-containing atmosphere. The $CO_2$ concentration is, for example, about 2% to 5%. The culture period can be set to a period long enough to allow formation of the alveolar organoid. Since long-term culture does not cause any particular problem, the culture period is not limited to a particular period, and may be, for example, 5 days or more, 6 days or more, 7 days or more, 8 days or more, 9 days or more, 10 days or more, 11 days or more, 12 days or more, 13 days or more, 14 days or more, or even more days, preferably 14 days or more, and more preferably about 14 days.

<Pulmonary Acinus Organoid>

In another aspect, the present disclosure provides a pulmonary acinus organoid. The present disclosure provides a pulmonary acinus organoid including two or more sac-like cellular structures, wherein each of the cellular structures includes a lumen and two cell layers at least partially covering the lumen, each of the two cell layers includes alveolar epithelial cells, fibroblasts, and/or vascular endothelial cells, and the lumen of one of the cellular structures is in communication with the lumen of the at least one other cellular structure. The pulmonary acinus organoid of the present disclosure has, for example, a structure in which lumens of a plurality of alveolar-like structures are in communication with each other, and thus has a structure similar to that of a pulmonary acinus in vivo. Accordingly, the pulmonary acinus organoid of the present disclosure enables simulative examination of the function of a pulmonary acinus, for example.

Each of the cell layers includes, for example, a lumen-side cell layer and a cell layer laminated on the lumen-side cell layer. It is preferable that the laminated cell layer is formed on the outer peripheral surface of the lumen-side cell layer, i.e., the laminated cell layer is formed on the side opposite to the lumen side so as to extend along the circumferential direction. In this case, the laminated cell layer is also referred to as an outer peripheral cell layer. When the cell layer includes the lumen-side cell layer and the outer peripheral cell layer, the lumen-side cell layer is preferably composed of alveolar epithelial cells, for example. Also, the outer peripheral cell layer is preferably composed of fibroblasts and/or vascular endothelial cells. The outer peripheral cell layer is laminated so as to partially or entirely cover the lumen-side cell layer, for example. The cell layers may cover the lumen so as to or so as not to allow the lumen to be in communication with the outside of the cell layers. That is, the cell layers may be formed so as to entirely cover the lumen or partially cover the lumen.

Examples of the alveolar epithelial cells include alveolar epithelial type I cells, alveolar epithelial type II cells, ciliated airway epithelial cells, goblet cells, club cells, bronchiolar epithelial cells, neuroendocrine cells, and basal cells.

The alveolar epithelial type II cells are, for example, cells that are positive for SFTPA, SFTPB, SFTPC, SFTPD, EpCAM, ABCA3, DCLAMP, CEACAM6, SLC34A2, and/or LPCAT1.

Examples of the fibroblasts include lung fibroblasts. The lung fibroblasts are, for example, cells that are positive for vimentin, ACTA2, COL1A1, ELN, FN, PDGFRA, POSTN, and/or TAGLN.

The outer peripheral cell layer may include, for example, vascular endothelial cells. Further, when the pulmonary acinus organoid includes alveolar epithelial cells, fibroblasts, and vascular endothelial cells, the pulmonary acinus organoid may include, for example, three cell layers partially covering the lumen. In this case, the three cell layers include an inner layer (lumen-side cell layer), an intermediate layer, and an outer layer (outer peripheral cell layer) in this order, and it is preferable that the inner layer is composed of the alveolar epithelial cells, the intermediate layer is composed of the fibroblasts, and the outer layer is composed of the vascular endothelial cells. The intermediate layer can also be referred to as a layer between the inner layer and the outer layer. The outer layer is laminated, for example, so as to partially or entirely cover the intermediate layer. The intermediate layer is laminated, for example, so as to partially or entirely cover the inner layer.

The cellular structure may have apical polarity on its lumen side or on the outer side, for example.

The pulmonary acinus organoid of the present disclosure includes, for example, a plurality of vesicle-like cellular structures that are in communication with each other internally. Accordingly, the pulmonary acinus organoid of the present disclosure can also be referred to as, for example, an acinar cellular structure in which respective vesicles are in communication with each other internally.

The above-described cellular structure may be, for example, an alveolar-like structure. The alveolar-like structure may be, for example, a structure having functions equivalent to those of an alveolus or an alveolar organoid. For the alveolar-like structure, reference can be made to, for example, the above descriptions regarding the sac-like cellular structure included in the structure of the present disclosure.

<Method for Producing Pulmonary Acinus Organoid>

In still another aspect, the present disclosure provides a method for producing a pulmonary acinus organoid. The present disclosure also provides a method for producing a pulmonary acinus-like organoid, including the step of culturing two or more spheroids in the presence of a pulmonary acinus organoid differentiation-inducing factor for inducing differentiation into pulmonary acinus organoids, wherein the spheroids are each composed of lung progenitor cells, lung fibroblasts, and/or vascular endothelial cells.

The alveolar organoid can be induced, for example, by culturing, preferably in a three-dimensional environment, the alveolar progenitor cells in the presence of an alveolar epithelial cell-inducing factor (induction step). The induction step can be performed, for example, by culturing the lung progenitor cells in the presence of a medium containing the alveolar epithelial cell-inducing factor. The lung progenitor cells are preferably CPM-positive lung progenitor cells. The lung progenitor cells are, for example, isolated lung progenitor cells and preferably isolated CPM-positive lung progenitor cells.

The induction step is performed, for example, using a medium containing the alveolar organoid differentiation-inducing factor. For the medium, reference can be made to the examples of the medium given above. The medium used in the induction step is preferably Ham's F12 medium containing B-27 Supplement.

There is no particular limitation on the number of spheroids as long as it is possible to construct a pulmonary acinus organoid. The number of spheroids is, for example, 2 to 150, 2 to 100, or 5 to 50, and preferably 5 to 50 from the viewpoint of ease in puncturing with the tubular body and allowing the internal cellular structure to be maintained properly.

The distance between the spheroids at the start of culture is, for example, 0 to 5000 $\mu$m, 0 to 2000 $\mu$m, 0 to 1500 $\mu$m, 1 to 1000 $\mu$m, 1 to 500 $\mu$m, 5 to 500 $\mu$m, or 10 to 150 $\mu$m. When the spheroids are in relatively close proximity to each other at the start of culture, lumens of pulmonary acinus organoids formed from these proximate spheroids tend to be in communication with each other relatively easily during the formation of the pulmonary acinus organoid. The distance between the spheroids can be adjusted, for example, by using a medium containing an extracellular matrix such as Matrigel, i.e., a viscous medium, and adjusting the seeding density in the medium.

The pulmonary acinus organoid differentiation-inducing factor is not limited to a particular factor as long as it is capable of inducing differentiation into pulmonary acinus organoids. The pulmonary acinus organoid differentiation-inducing factor is, for example, a TGF-$\beta$1 inhibitor, BMP inhibitor, steroid agent, phosphodiesterase inhibitor, KGF (Keratinocyte Growth Factor), Wnt activator, GSK3$\beta$ inhibitor, ROCK inhibitor, MAP kinase inhibitor, EGF (Epidermal Growth Factor), and/or FGF. Of these, the TGF-B1 inhibitor and BMP inhibitor are preferable. One type of pulmonary acinus organoid differentiation-inducing factor may be used, and two or more types of pulmonary acinus organoid differentiation-inducing factors may be used in combination. By using the TGF-$\beta$1 inhibitor and/or the BMP inhibitor as the pulmonary acinus organoid differentiation-inducing factor, preferably by using both the TGF-$\beta$1 inhibitor and the BMP inhibitor as the pulmonary acinus organoid differentiation-inducing factors, the method for producing the pulmonary acinus organoid according to the present disclosure can efficiently promote the adhesion and fusion between the spheroids, thereby promoting the communication between the lumens of alveoli formed from the respective spheroids, for example.

The TGF-B1 inhibitor refers to a substance capable of inhibiting TGF-B1 (transforming growth factor-$\beta$ superfamily type 1, transforming growth factor-$\beta$1). Examples of the TGF-$\beta$1 inhibitor include: compounds such as SB 431542 (4-(4-(benzo[d][1,3]dioxol-5-yl)-5-(pyridin-2-yl)-1H-imidazol-2-yl)benzamide), sc-203294 (2-(5-chloro-2-fluorophenyl)pteridin-4-yl)pyridin-4-yl amine), A83-01 (3-(6-methyl-2-pyridinyl)-N-phenyl-4-(4-quinolinyl)-1H-pyrazole-1-carbothioamide), LDN 193189 (4-[6-[4-(1-piperazinyl)phenyl]pyrazolo[1,5-a]pyrimidin-3-yl]quinoline), SB 505124 (2-[4-(1,3-benzodioxol-5-yl)-2-(1,1-dimethylethyl)-1H-imidazol-5-yl]-6-methyl-pyridine), SD-208 (2-(5-Chloro-2-fluorophenyl)pteridin-4-yl]pyridin-4-yl-amine), and SB-525334 (6-[2-tert-butyl-5-(6-methyl-pyridin-2-yl)-1H-imidazol-4-yl]-quinoxaline); and TGF-$\beta$1 binding peptides such as PG-002 (PeptiGrowth Inc.). Of these, SB 431542 is preferable. Also, the TGF-$\beta$1 inhibitor may be, for example, a nucleic acid (e.g., siRNA) that suppresses the expression of the gene encoding such a protein or the gene encoding the receptor for such a protein.

The TGF-$\beta$1 inhibitor concentration in the medium is not limited to a particular concentration, and is, for example, 100 nM to 100 $\mu$M, specifically 500 nM to 90 $\mu$M, 1 $\mu$M to 50 $\mu$M, 5 $\mu$M to 25 $\mu$M, or 10 $\mu$M to 20 $\mu$M, and preferably about 10 $\mu$M.

The BMP inhibitor refers to a substance capable of inhibiting BMP (bone morphogenetic protein, bone morphogenetic factor). Examples of the BMP inhibitor include: BMP-binding proteins such as Noggin, Chordin, Gremlin, Follistatin, Inhibin, Twisted Gastrulation, Coco, and DAN; BMP-binding peptides such as PG-004 (PeptiGrowth Inc.); and compounds such as K02288 (3-[6-amino-5-(3,4,5-trimethoxyphenyl)-3-pyridinyl]-phenol), Dorsomorphin (6-[4-[2-(1-piperidinyl)ethoxy]phenyl]-3-(4-pyridinyl)pyrazolo[1,5-a]pyrimidine), LDN-193189 (4-[6-[4-(1-piperazinyl)phenyl]pyrazolo[1,5-a]pyrimidin-3-yl]quinoline dihydrochloride), LDN-212854 (5-[6-[4-(1-piperazinyl)phenyl] pyrazolo[1,5-a]pyrimidin-3-yl]-quinoline), LDN-214117 (1-(4-(6-Methyl-5-(3,4,5-trimethoxyphenyl)pyridin-3-yl)phenyl) piperazine), ML347 (5-[6-(4-Methoxyphenyl)pyrazolo[1,5-a]pyrimidin-3-yl]-quinoline), DMH1 (4-(6-(4-Isopropoxyphenyl)pyrazolo[1,5-a]pyrimidin-3-yl)quinoline), and DMH2 (4-[6-[4-[2-(4-Morpholinyl)ethoxy]phenyl]pyrazolo[1,5-a]pyrimidin-3-yl]-quinoline). Of these, Noggin is preferable. Also, the BMP inhibitor may be, for example, a nucleic acid (e.g., siRNA) that suppresses the expression of the gene encoding such a protein or the gene encoding the receptor for such a protein.

The BMP inhibitor concentration in the medium is not limited to a particular concentration, and is, for example, 100 pg/ml to 100 $\mu$g/ml, specifically 500 pg/ml to 50 $\mu$g/ml, 1 ng/ml to 25 $\mu$g/ml, 10 ng/ml to 10 $\mu$g/ml, or 100 ng/ml to 1 $\mu$g/ml, and preferably about 100 ng/ml.

The steroid agent is a steroidal anti-inflammatory drug. The steroid agent is, for example, glucocorticoid or a synthetic derivative thereof. Specific examples of the steroid agent include hydrocortisone, hydrocortisone succinate, prednisolone, methylprednisolone, methylprednisolone succinate, triamcinolone, triamcinolone acetonide, dexamethasone, and betamethasone. Of these, dexamethasone or hydrocortisone is preferable.

The steroid agent concentration in the medium is not limited to a particular concentration, and is, for example, 1 nM to 1 $\mu$M, specifically 5 nM to 900 nM, 10 nM to 800 nM, 20 nM to 700 nM, 30 nM to 600 nM, 40 nM to 500 nM, 50 nM to 400 nM, 60 nM to 300 nM, 70 nM to 200 nM, or 80 nM to 100 nM, and preferably about 50 nM.

The phosphodiesterase inhibitor refers to a compound that increases the intracellular concentration of cAMP or cGMP by inhibiting phosphodiesterase (PDE). Examples of the phosphodiesterase inhibitors include 1,3-Dimethylxanthine, 6,7-Dimethoxy-1-(3,4-dimethoxybenzyl)isoquinoline, 4-{[3',4'-(Methylenedioxy)benzyl]amino}-6-methoxyquinazoline, 8-methoxymethyl-3-isobutyl-1-methylxanthine, and 3-isobutyl-1-methylxanthine (IBMX). Of these, 1,3-Dimethylxanthine or IBMX is preferable.

The phosphodiesterase inhibitor concentration in the medium is not limited to a particular concentration, and is, for example, 1 nM to 1 $\mu$M, and specifically 5 nM to 900 nM, 10 nM to 800 nM, 20 nM to 700 nM, 30 nM to 600 nM, 40 nM to 500 nM, 50 nM to 400 nM, 60 nM to 300 nM, 70 nM to 200 nM, or 80 nM to 100 nM.

The KGF is a protein encoded by the polynucleotide registered under NCBI Accession Number NM_002009. The KGF may be in a state of being activated by cleavage with a protease.

The KGF concentration in the medium is, for example, 10 ng/ml to 1 $\mu$g/ml, 20 ng/ml to 900 ng/ml, 30 ng/ml to 800 ng/ml, 40 ng/ml to 700 ng/ml, 50 ng/ml to 600 ng/ml, 60 ng/ml to 500 ng/ml, 70 ng/ml to 400 ng/ml, 80 ng/ml to 300 ng/ml, or 90 ng/ml to 200 ng/ml.

Examples of the Wnt activator include CHIR99021, Rspo3, and Wnt3a.

The Wnt activator concentration in the medium is not limited to a particular concentration, and is, for example, 1 nM to 100 $\mu$M, 10 nM to 90 $\mu$M, 10 nM to 50 $\mu$M, 20 nM to 10 $\mu$M, 30 nM to 1 $\mu$M, 40 nM to 500 nM, 50 nM to 400 nM, 60 nM to 300 nM, 70 nM to 200 nM, or 80 nM to 100nM. When Rspo3 is used as the Wnt activator in the medium, the Rspo3 concentration is 100 ng/ml to 5 $\mu$g/ml, 200 ng/ml to 4 $\mu$g/ml, or 500 ng/ml to 1 $\mu$g/ml. When Wnt3a is used as the Wnt activator in the medium, the Wnt3a concentration is 10 ng/ml to 1 $\mu$g/ml, 20 ng/ml to 800 ng/ml, or 100 ng/ml to 500 ng/ml.

The GSK3$\beta$ inhibitor is a substance that inhibits the kinase activity (e.g., the ability to phosphorylate $\beta$-catenin) of the GSK-3$\beta$ protein. Examples of the GSK3$\beta$ inhibitor include: indirubin derivatives such as BIO (GSK-3$\beta$ inhibitor IX; 6-bromoindirubin-3'-oxime); maleimide derivatives such as SB216763 (3-(2,4-dichlorophenyl)-4-(1-methyl-1H-indol-3-yl)-1H-pyrrole-2,5-dione); phenyl $\alpha$-bromomethyl ketone compounds such as GSK-3$\beta$ inhibitor VII (4-dibromoacetophenone); cell membrane-permeable phosphorylated peptides such as L803-mts (GSK-3$\beta$ peptide inhibitor; Myr-N-GKEAP-PAPPQSpP-NH$_2$ (SEQ ID NO: 1)); CHIR99021 (6-[2-[4-(2,4-dichlorophenyl)-5-(4-methyl-1H-imidazol-2-yl)pyrimidin-2-ylamino]ethylamino]pyridine-3-carbonitrile); and nucleic acid molecules (such as siRNA, shRNA, and an antisense) that suppress the expression of the GSK3$\beta$ protein. As the GSK3$\beta$ inhibitor, CHIR99021 is preferable in terms of its high selectivity for the GSK3$\beta$ protein.

The GSK3$\beta$ inhibitor concentration in the medium is, for example, 1 nM to 100 $\mu$M, 10 nM to 100 $\mu$M, 10 nM to 800 nM, 20 nM to 700 nM, 30 nM to 600 nM, 40 nM to 500 nM, 50 nM to 400 nM, 60 nM to 300 nM, 70 nM to 200 nM, or 80 nM to 100 nM.

The ROCK inhibitor is a substance capable of suppressing the function of Rho-kinase (ROCK). Examples of the ROCK inhibitor include Y-27632 ((+)-(R)-trans-4-(1-aminoethyl)-N-(4-pyridyl)cyclohexanecarboxamide dihydrochloride), Fasudil/HA1077 (5-(1,4-Diazepane-1-sulfonyl)isoquinoline), H-1152 ((S)-(+)-2-Methyl-1-[(4-methyl-5-isoquinolinyl)sulfonyl]homopiperazine), Wf-536 ((+)-(R)-4-(1-Aminoethyl)-N-(4-pyridyl) benzamide), and nucleic acid molecules (such as siRNA, shRNA, and an antisense) that suppress the expression of the ROCK protein. Of these, Y-27632 is preferable.

The ROCK inhibitor concentration in the medium is not limited to a particular concentration, and is, for example, 1 nmol/l to 50 $\mu$mol/l, 10 nmol/l to 40 $\mu$mol/l, 50 nmol/l to 30 $\mu$mol/l, 100 nmol/l to 25 $\mu$mol/l, 500 nmol/l to 20 $\mu$mol/l, or 750 nmol/l to 15 $\mu$mol/l.

Examples of the MAP kinase inhibitor include SB 203580 (4-(4-Fluorophenyl)-2-(4-methylsulfinylphenyl)-5-(4-pyridyl)1H-imidazole).

The MAP kinase inhibitor concentration in the medium is not limited to a particular concentration, and is, for example, 1 nM to 100 $\mu$M, 10 nM to 100 $\mu$M, 10 nM to 800 nM, 20 nM to 700 nM, 30 nM to 600 nM, 40 nM to 500 nM, 50 nM to 400 nM, 60 nM to 300 nM, 70 nM to 200 nM, or 80 nM to 100 nM.

The EGF is a protein encoded by the polynucleotide registered under NCBI Accession Number NM_001178130, NM_001178131, NM_001963, or NM_001357021.

The EGF concentration in the medium is, for example, 1 ng/ml to 1 $\mu$g/ml, 10 ng/ml to 1 $\mu$g/ml, 20 ng/ml to 1 $\mu$g/ml, 30 ng/ml to 900 ng/ml, 40 ng/ml to 800 ng/ml, 50 ng/ml to 700 ng/ml, 60 ng/ml to 600 ng/ml, 70 ng/ml to 500 ng/ml, 80 ng/ml to 400 ng/ml, or 90 ng/ml to 300 ng/ml.

Examples of the FGF include FGF2 and FGF10. The FGF2 is a protein encoded by the polynucleotide registered under NCBI Accession Number NM_001361665. The FGF10 is a protein encoded by the polynucleotide registered under NCBI Accession Number NM_004465. The FGF may be in a state of being activated by cleavage with a protease.

The FGF concentration in the medium is not limited to a particular concentration, and is, for example, 1 ng/ml to 1 $\mu$g/ml, 10 ng/ml to 1 $\mu$g/ml, 20 ng/ml to 1 $\mu$g/ml, 30 ng/ml to 900 ng/ml, 40 ng/ml to 800 ng/ml, 50 ng/ml to 700 ng/ml, 60 ng/ml to 600 ng/ml, 70 ng/ml to 500 ng/ml, 80 ng/ml to 400 ng/ml, or 90 ng/ml to 300 ng/ml.

The medium may contain, for example, any of the above-described extracellular matrices such as Matrigel.

The number of days in culture in the step of inducing the pulmonary acinus organoid can be set depending on the period required for inducing the pulmonary acinus organoid. The lower limit of the number of days in culture is, for example, 2 days or more, 3 days or more, 4 days or more, 5 days or more, 6 days or more, 7 days or more, 8 days or more, 9 days or more, 10 days or more, or even more days. The upper limit of the number of days in culture is, for example, 35 days or less, 30 days or less, 25 days or less, 20 days or less, 15 days or less, 10 days or less, or 8 days or less. Specifically, the number of days in culture is 2 to 14 days or 3 to 8 days, for example.

The culture conditions used in the step of inducing the pulmonary acinus organoid are not limited to particular conditions, and may be set, for example, based on the culture conditions for the pulmonary acinus organoid. Specifically, the culture temperature is, for example, about 30°C to 40°C and preferably about 37°C. The culture is performed, for example, in a $CO_2$-containing atmosphere. The $CO_2$ concentration is, for example, about 2% to 5%.

The structure of the present disclosure can be used suitably for, for example, evaluation of the effect, toxicity, and the like of an evaluation target component on cells of an organ or internal organ of interest and evaluation of the metabolism, dynamics, and the like of an evaluation target component in cells of an organ or internal organ of interest.

<Method for Evaluating Structure>

In still another aspect, the present disclosure provides an evaluation method using a cellular structure. The present disclosure provides an evaluation method using the structure of the present disclosure, including the steps of: preparing a coexistence system in which a test substance and the cellular structure are present together (preparation step); and evaluating the cellular structure and/or the test substance present in the coexistence system (evaluation step). Therefore, according to the evaluation method of the present disclosure, it is possible to evaluate the effect of a test substance on the cellular structure, for example, by introducing the test substance into the lumen of the cellular structure via the tubular body. According to the evaluation method of the present disclosure, it is possible to evaluate the dynamics of the test substance, for example, by introducing the test substance into the lumen of the cellular structure via the tubular body.

Examples of the test substance include: low molecular weight compounds; proteins such as antibodies, cytokines, and chemokines; carbohydrate chains; nucleic acids; microorganisms such as bacteria; viruses; air conditioner refrigerants; environmental particulate matter such as PM2.5, carbon nanofibers, dust, asbestos, silica, microplastics, and exhaust particulate matter; and gases such as oxygen, carbon dioxide, and air.

In the preparation step, a coexistence system in which the test substance and the cellular structure are present together is prepared. Specifically, the coexistence step is performed by creating a state where the test substance and the cellular structure can come into contact with each other. Thus, in the preparation step, for example, when the test substance acts on the cellular structure or when the cellular structure acts on the test substance, a change is caused in the cellular structure and/or the test substance. The contact may be achieved, for example, by adding the test substance to a medium containing the cellular structure or by introducing the test substance into the lumen of the cellular structure via the tubular body of the structure, with the latter being preferred. In the preparation step, the coexistence system is cultured, for example. For the culture medium and other components used for the above-described culture, reference can be made to the above descriptions thereon, for example.

The conditions for coexistence in the preparation step may be set as appropriate, for example, according to the culture conditions for the cellular structure. Specifically, the temperature in the coexistence step is, for example, 30°C to 40°C, 35°C to 38°C, or about 37°C. The coexistence step is performed for, for example, 10 minutes to 48 hours, 15 to 24 hours, or 2 to 12 hours.

Next, in the evaluation step, evaluation is performed for the cellular structure and/or the test substance after the preparation step. Specifically, in the evaluation step, for example, the change in the cellular structure and/or the change in the test substance after the preparation step is evaluated. In the evaluation step, evaluation is performed for one or more items.

The evaluation of the cellular structure can be performed, for example, by evaluating the shape, the hardness, the ratio of contraction or expansion, a cell differentiation marker, a fibrosis marker, an aging marker, an oxidative stress marker, or an apoptosis marker. The shape of the cellular structure can be evaluated, for example, by evaluating the change in the cellular structure using a microscope. The microscope may be, for example, an optical microscope such as a phase-contrast microscope or a fluorescence microscope. The hardness of the cellular structure can be evaluated, for example, by applying pressure to the cellular structure under observation with the microscope and evaluating whether the structure of the cellular structure is maintained. The ratio of contraction or expansion of the cellular structure can be evaluated, for example, by evaluating the change ratio in size when pressure or reduced pressure is applied to the lumen of the cellular structure via the tubular body. Evaluation of a marker such as a cell differentiation marker, a fibrosis marker, an aging marker, an oxidative stress marker, or an apoptosis marker of the cellular structures can be performed, for example, by evaluating whether the presence or absence of the expression of the marker or the expression level of the marker in the cellular structure. The marker can be set as appropriate, for example, according to the type of the cells constituting the cellular structure.

The test substance can be evaluated, for example, by evaluating the concentration of the test substance, the concentration of a metabolite of the test substance, or the partial pressure of a gas. The concentration of the test substance can be evaluated, for example, by evaluating a change in the concentration of the test substance introduced into the lumen of the cellular structure and/or evaluating the concentration of the test substance introduced into the lumen of the cellular structure and the concentration of the test substance outside the cellular structure. In the latter case, by evaluating the test substance, the dynamics of the test substance in the cellular structure can be evaluated in the evaluation step. The concentration of a metabolite of the test substance can be evaluated, for example, by evaluating a change in the concentration of the metabolite of the test substance introduced into the lumen of the cellular structure and/or evaluating the concentration of the metabolite of the test substance introduced into the lumen of the cellular structure and the concentration of the metabolite of the test substance outside the cellular structure. In the latter case, by evaluating the metabolite of the test substance, the dynamics of the metabolite of the test substance in the cellular structure can be evaluated in the evaluation step. The partial pressure of a gas can be evaluated, for example, by evaluating a change in the partial pressure of the test substance (gas) introduced into the lumen of the cellular structure.

In the evaluation method of the present disclosure, the test substance may be, for example, subjected to screening based on the evaluation result obtained in the evaluation step. Examples

Next, examples of the present invention will be described. It is to be noted, however, that the present invention is not limited by the following examples. Commercially available reagents in the examples were used in accordance with their protocols, unless otherwise stated.

Example 1

Expansion and contraction of alveolar organoids were measured using a device of the present disclosure.

(1) Preparation of lung fibroblasts

Prior to the measurement of expansion and contraction of alveolar organoids using a device of the present disclosure, alveolar organoids were constructed. Specifically, first, lung fibroblasts were prepared. As a lung fibroblast culture medium, DMEM (Low Glucose) (manufactured by FUJIFILM Corporation) medium containing 10% FBS (FB-1365/500, manufactured by Biosera) was prepared. Next, thawed lung fibroblasts were cultured using the above-described lung fibroblast culture medium on a 10-cm dish at 37°C and 5% $CO_2$ for one week. During the culture, the medium was replaced every 2 to 3 days. After the culture, the lung fibroblast culture medium was removed from the dish, and 3 ml of PBS (phosphate buffered saline, manufactured by Cell Science & Technology Institute, Inc.) was added for washing. After the washing, the PBS was removed from the dish, and then, 3 ml of 0.1% trypsin/EDTA prepared by mixing 2.5 g/l-trypsin/1 mmol/l-EDTA solution (manufactured by Nacalai Tesque, Inc.) and 0.5 mol/l EDTA/PBS at a ratio of 2:3 was added to the dish. After the addition, the dish was left to stand still in an incubator at 37°C and 5% $CO_2$ for 3 minutes, thereby dissociating the lung fibroblasts from the dish. After the dissociation, 3 ml of the lung fibroblast culture medium was added, and the fibroblasts were collected into a 50-ml centrifuge tube. After the collection, the fibroblasts were centrifuged at 120 × g at room temperature (about 24°C, hereinafter the same) for 5 minutes. After the centrifugation, the supernatant was removed, and the lung fibroblasts were resuspended in 3 ml of the lung fibroblast culture medium. After the resuspension, the lung fibroblasts were left to stand still on ice.

(2) Preparation of CPM-positive lung progenitor cells

Next, CPM-positive lung progenitor cells were prepared. Specifically, human iPS cells (induced pluripotent stem cells) were cultured for 21 days to differentiate them into lung progenitor cells. After the differentiation, the cells were washed once with PBS, and 0.5 mol/l EDTA (manufactured by Nacalai Tesque, Inc.) was added. After the addition, the culture was left to stand still in an incubator at 37°C and 5% $CO_2$ for 12 minutes. After the still standing, the supernatant was removed, Accutase (manufactured by Innovative Cell Technologies) was added, and the culture was left to stand still in an incubator at 37°C and 5% $CO_2$ for 30 minutes. After the still standing, the lung progenitor cells were collected and suspended twice using 2% FBS/DMEM containing 1000-fold diluted Y-27632 (10 mmol/l). Thereafter, the cell suspension was filtered through a 40-$\mu$m strainer (EASY strainer™, manufactured by Greiner) to collect a cell suspension containing only single cells. After the collection, the cell suspension was centrifuged at 120 $\times$ g at 4°C for 5 minutes. After the centrifugation, the supernatant was removed by aspiration, and FACS buffer (1% BSA/PBS supplemented with 1000-fold diluted Y-27632 (10 mmol/l)) was added. After the addition, the cell suspension was centrifuged for 1 minute using a bench-top centrifuge, and after the centrifugation, the supernatant was removed. After the removal, the cells were washed with FACS buffer, and after the washing, FACS buffer containing 200-fold diluted CPMAb (manufactured by FUJIFILM Wako Pure Chemical Corporation) was added. After the addition, primary staining was performed on ice for 20 minutes with tapping every 5 minutes. After the primary staining, the cells were washed with FACS buffer, and after the washing, FACS buffer containing 500-fold diluted Alexa Fluor® 647-donkey anti-mouse IgG (manufactured by Thermo Fisher Scientific) was added. After the addition, secondary staining was performed on ice for 30 minutes under light-shielded conditions. After the secondary staining, the cells were washed twice with FACS buffer, and a cell suspension containing the lung progenitor cells at a concentration of approximately $5 \times 10^7$ cells/ml was prepared using FACS buffer. After the preparation, the cell suspension was then aliquoted into tubes dedicated for a cell sorter (manufactured by Cell Sorter SH800S, Sony Corporation) at 250 $\mu$l/tube. Thereafter, CPM-positive lung progenitor cells were selectively collected using the cell sorter. The collected CPM-positive lung progenitor cells were cryopreserved, and in the following items (5), (9), and (10) of Example 1, they were used after thawing.

(3) Construction of spheroids

Spheroids were constructed using the CPM-positive lung progenitor cells and the lung fibroblasts prepared in the above item (1) or (2) of Example 1. Specifically, the CPM-positive lung progenitor cells and the lung fibroblasts were mixed together, and the resultant mixture was centrifuged at 800 $\times$ g for 5 minutes at room temperature. After the centrifugation, the supernatant was removed, and the CPM-positive lung progenitor cells and the lung fibroblasts were suspended in a seeding medium such that both the cells were present at a concentration of $1 \times 10^6$ cells/ml. In the following items (5), (9), and (10) of Example 1, the CPM-positive lung progenitor cells and the lung fibroblasts were suspended in the seeding medium such that the CPM-positive lung progenitor cells were present at $2.4 \times 10^6$ cells/ml and the lung progenitor cells were present at $1.6 \times 10^6$ cells/ml. The seeding medium was prepared by adding Bovine Albumin Fraction V (7.5% solution, manufactured by GIBCO), 1.5 mmol/l HEPES (manufactured by SIGMA), 0.8 mmol/l $CaCl_2$, 0.1% ITS, 50 nmol/l Dexamethasone, 100 $\mu$mol/l IBMX, 0.5% penicillin/streptomycin (manufactured by FUJIFILM Wako Pure Chemical Corporation), and 1% B27 supplement (manufactured by GIBCO) to Ham's F12 medium (manufactured by FUJIFILM Wako Pure Chemical Corporation) to prepare a 3D medium, and then adding 10000-fold diluted Recombinant Human KGF (100 $\mu$g/ml, FGF-7, KGF, manufactured by Peprotech), 1000-fold diluted 8-Br-cAMP (0.1 mol/l, manufactured by Biolog Life Science Institute), and 1000-fold diluted Y-27632 (10 mmol/l, manufactured by LCL Laboratories) to the thus-prepared 3D medium. After the suspension and prior to the cell seeding, 500 $\mu$l of the seeding medium was added to each well of a 24-well plate (Corning® Elplasia® Multiwell Plate 24-well, manufactured by Corning), and the plate was centrifuged at 100 $\times$ g for 2 minutes at room temperature to remove air bubbles. The maintenance medium was prepared by adding 10000-fold diluted Recombinant Human KGF (100 $\mu$g/ml, FGF-7, KGF, manufactured by Peprotech) and 1000-fold diluted 8-Br-cAMP (0.1 mol/l, manufactured by Biolog Life Science Institute) to the 3D medium. After the centrifugation, the cell suspension was added to each well of the plate at 500 $\mu$l per well, thereby seeding the cells at $1 \times 10^6$ cells per well. After the seeding, the medium was replaced every 2 to 3 days, and the cells were cultured for 6 days. In the medium replacement, to reduce the influence on the spheroids, partial replacement of the maintenance medium was performed twice (500 $\mu$l of the maintenance medium was replaced in each replacement). After the culture, it was confirmed that the seeded cells had aggregated and that spheroids of the CPM-positive lung progenitor cells and the lung fibroblasts had been constructed.

(4) Construction of alveolar organoids

Next, alveolar organoids were constructed using the spheroids obtained in the above item (3) of Example 1. The spheroids were collected from the wells into a 2 ml tube, and 500 $\mu$l of the maintenance medium was re-added to the wells after the collection. After the re-addition, gentle pipetting was performed to ensure that no spheroids remained in the wells, and thereafter, the spheroids were collected again. After the collection, the supernatant was removed, and the tube was left to stand still on ice. After the still standing, 35 $\mu$l of Matrigel® Growth Factor Reduced (Matrigel, manufactured by Corning) was added, and the spheroids were quickly suspended to prevent air from entering. After suspending the spheroids, the suspension was dispensed onto a 35 mm dish so as to form dome-shaped 50 $\mu$l aliquots, thereby providing the spheroids embedded in 50% to 70% Matrigel. Alternatively, after the still standing, a 35 mm dish was placed on a LABOPAD C (manufactured by TOSC Japan Ltd.) set at 0°C, and Matrigel was dispensed onto the dish so as to form ten dome-shaped aliquots of 5 to 7 $\mu$l. After dispensing the Matrigel, one of the spheroids collected under a microscope and 3 to 5 $\mu$l of the maintenance medium were added to each aliquot of the Matrigel, thereby providing the spheroids embedded in 50% to 70% Matrigel. In the process of embedding the spheroids in the Matrigel, in order to prevent air from entering and keep the cell density per area low, care was taken to perform dripping in a subtle circular motion. After the embedding process, the Matrigel was placed in an incubator at 37°C and 5% $CO_2$ for about 15 to 30 minutes, whereby the Matrigel turned into gel. After the gelation, 2 ml of the maintenance medium was added along the wall. After the addition, medium replacement using the above-described maintenance medium was performed every 2 to 3 days, and the spheroids were cultured for 3 to 8 days in an incubator. After the culture, it was confirmed that alveolar organoids had been constructed.

(5) Measurement of alveolar organoids

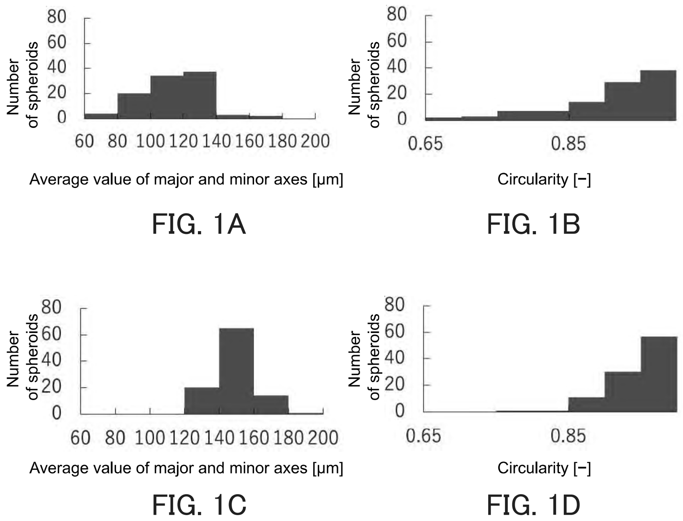

Next, the size of the spheroids on day 6 of culture and immediately before being embedded in the Matrigel (obtained in the above item (3) of Example 1), the size of the alveolar organoids after being embedded in Matrigel (obtained in the above item (4) of Example 1), and the thickness of the alveolar organoids were measured. The measurements were performed by capturing images and analyzing the images using an inverted microscope (manufactured by Olympus Corporation) and imaging software cellSens (manufactured by Olympus Corporation). The size of the spheroids was calculated by approximating each spheroid in an image captured by the inverted microscope to an ellipse and calculating the length of the major axis $d_L$ and the length of the minor axis $d_S$. Also, using the length of the major axis $d_L$ and the length of the minor axis $d_S$, the circularity C was calculated as per the equation (1), and the thus-determined circularity C was used as the degree of approximation to a perfect circle. The results thereof are shown in FIG. 1.

$$C = d_S/d_L \ ... \ (1)$$

    C: Circularity
    $d_S$: Length of minor axis
    $d_L$: Length of major axis

FIG. 1 shows graphs showing the average value of the major and minor axes of spheroids and graphs showing the circularity of spheroids. FIG. 1A shows the average value of the major and minor axes of spheroids induced from $1 \times 10^6$ cells, FIG. 1B shows the circularity of spheroids induced from $1 \times 10^6$ cells, FIG. 1C shows the average value of the long axis and short axis of spheroids induced from $2 \times 10^6$ cells, and FIG. 1D shows the circularity of spheroids induced from $2 \times 10^6$ cells. In FIGs. 1A and 1C, the horizontal axis indicates the average value of the major and minor axes, and the vertical axis indicates the number of organoids. In FIGs. 1B and 1D, the horizontal axis indicates the circularity, and the vertical axis indicates the number of organoids. It can be seen from FIGs. 1A and 1C that increasing the number of cells used for spheroid formation leads to an increase in the average value of the major and minor axes of the formed spheroids and also to suppression of variation in diameter of the spheroids. Further, it can be seen from FIGs. 1B and 1D that increasing the number of cells used for spheroid formation leads to an increase in the degree of circularity of the formed spheroids.

(6) Preparation of frozen sections of alveolar organoids

Frozen sections were prepared from the alveolar organoids obtained in the above item (4) of Example 1. Using a 1000-$\mu$l pipette tip with the end cut off, the culture containing the alveolar organoids was pipetted to dissociate the Matrigel. After the dissociation, excess medium was removed, and then, the alveolar organoids, together with the surrounding Matrigel, were immobilized using iPGell (manufactured by Genostaff). After the immobilization, 4% PFA (paraformaldehyde, manufactured by Muto Pure Chemicals Co., Ltd.) was added, and immobilization was performed at room temperature for 60 minutes. After the immobilization, the sample was washed three times with PBS for 5 minutes each, added to 20% sucrose/PBS, and then subjected to sucrose substitution overnight at 4°C. Thereafter, moisture was removed, and the sample was immersed in OCT Compound (manufactured by Sakura Finetek Japan Co., Ltd.) for 15 minutes. After the immersion, the sample was embedded in fresh OCT Compound and frozen using liquid nitrogen. After the freezing, the sample was cut at -25°C using a cryostat (Hyrax C25, Carl Zeiss Co. Ltd) to prepare frozen sections with a thickness of 8 $\mu$m.

(7) Preparation of tissue specimens of frozen sections of alveolar organoids

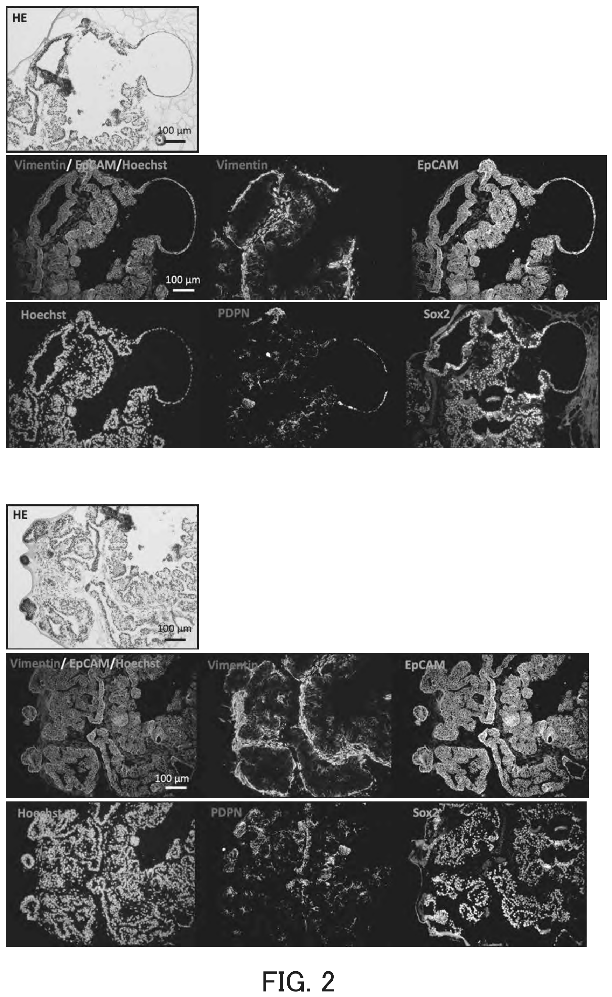

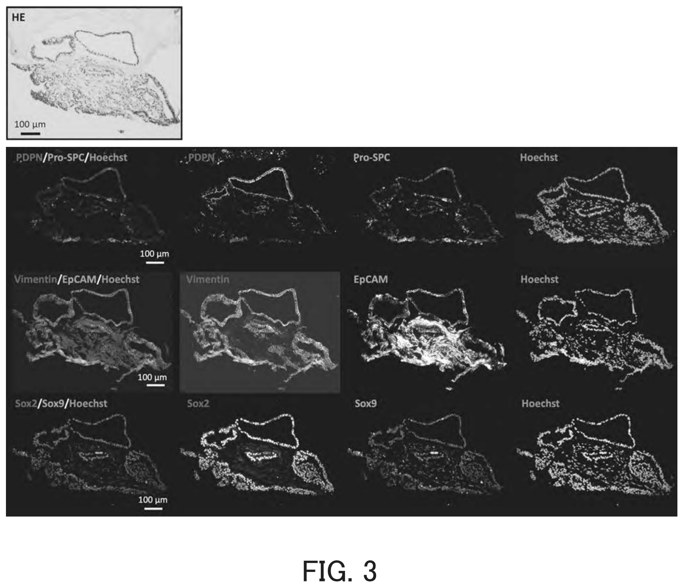

The frozen sections obtained in the above item (6) of Example 1 were subjected to HE staining to prepare tissue specimens. First, the frozen sections obtained in the above item (6) of Example 1 were immersed in water twice for 5 minutes each and then in hematoxylin for 5 minutes. Thereafter, the sections were immersed in water for 5 minutes and then in eosin for 5 minutes. Subsequently, in a draft chamber, the sections were immersed in ethanol three times for 5 minutes each and then in xylene three times for 5 minutes each, thereby optically clearing the tissue. After the optical clearing, the frozen sections were mounted on a 24 × 60C cover glass (manufactured by MATSUNAMI) using Entellan™ new (manufactured by Merck) and left to stand still for more than one night to encapsulate the frozen sections. Thereafter, the sections were washed three times with PBS for 5 minutes each, and 300 μl of 0.1% Triton X-100 (manufactured by Alfa Aesar)/PBS was added, followed by blocking for 20 minutes. After the blocking, a primary antibody diluted in 3% BSA (Bovine serum albumin, manufactured by Sigma Awsar)/PBS was added, and primary staining was performed overnight at 4°C. As the primary antibody, EpCAM (diluted 200-fold, #sc-66020, manufactured by SantaCruz), vimentin (diluted 500-fold, #sc-6260, manufactured by Santa Cruz), PDPN (diluted 200-fold, #17-9381-42, manufactured by eBioscience), Pro-SPC (diluted 3000-fold, #WRAB-9337, manufactured by Seven Hills Bioreagents), SOX2 (diluted 500-fold, #14-9811-82, manufactured by Invitrogen), or SOX9 (diluted 500-fold, #ab185230, manufactured by Abcam) was used. After the primary staining, the sections were washed three times with PBS for 5 minutes each, a secondary antibody diluted 400-fold in 3% BSA/PBS and Hoechst 33342 (manufactured by Thermo Fisher Scientific) diluted 1000-fold were added, and the sections were subjected to secondary staining for 1 hour under light-shielded conditions. As the secondary antibody, Alexa Fluor® 488 Phalloidin Conjugate or Alexa Fluor® 647 Phalloidin Conjugate was used. After the secondary staining, the sections were washed twice with PBS for 5 minutes each under light-shielded conditions, reacted with 4% PFA for 10 minutes, and then washed twice with PBS for 5 minutes each. After the washing, the frozen sections were mounted on a 24 × 60C cover glass using Fluoromount/Plus (manufactured by DBS) and then left to stand still for 1 hour to encapsulate the frozen sections. After the encapsulation, the sections were observed using a microscope (BX51WI, manufactured by Olympus). The results thereof are shown in FIGs. 2 and 3.

FIG. 2 shows photographs of images of stained alveolar organoids. In FIG. 2, the photographs show, from the left, HE-stained images, EpCAM/vimentin/-Hoechest-stained fluorescence images, PDPN/Hoechest-stained fluorescence images, and SOX2/Hoechest-stained fluorescence images. As can be seen from FIG. 2, it was confirmed that the alveolar organoid had a lumen-like complex three-dimensional structure and that alveolar epithelial type I cells, which play an important role for gas exchange in alveoli, were induced.

FIG. 3 shows photographs of stained images of alveolar organoids. In FIG. 3, the photographs show, from the left, a HE-stained image, a Pro-SPC/PDPN/Hoeches-stained fluorescence image, a Pro-SPC/Hoeches-stained fluorescence image, and a PDPN/Hoechest-stained fluorescence image. As can be seen from FIG. 3, it was confirmed that the alveolar organoid was constituted by tissue stem cells of the alveolar region and was able to maintain the differentiated state as alveolar epithelial type II cells, which are important in studying the pathogenesis of respiratory diseases and the pulmonary toxicity of drugs.

(8) Fabrication of puncture device

Next, a puncture device was fabricated. A 0-tube (TST 150-6, Septum Theta, manufactured by WORLD PRECISION INSTRUMENTS) or a glass tube with an outer diameter of 1 mm (manufactured by FUJISTON) was stretched using a puller (PC-10, manufactured by NARISHIGE), thus obtaining a glass tube with a tapered end. Thereafter, a glass sphere heated to 75°C was adhered to the glass tube using a microphage (MF-900, manufactured by NARISHIGE), and the tapered and closed end of the glass tube was opened by stopping the heating. After opening the end of the glass tube, a microcapillary (TSP100375 Fused Silica Capillary, manufactured by CM Scientific) was inserted into the hole at the end on the unstretched side of the glass tube. After the insertion, epoxy adhesive (ARALDITE RT30, manufactured by Huntsman Japan) was added to the tube on a silicon sheet, and the tube was left to stand still overnight to confirm that the adhesive had completely cured. Thus, the puncture device was fabricated.

(9) Construction of apparatus for fluid delivery and pressurization with respect to alveolar organoids using puncture device

An apparatus was constructed that performs fluid delivery and pressurization with respect to the alveolar organoids constructed in the above item (4) of Example 1 using the puncture device fabricated in the above item (8) of Example 1. First, a syringe pump (KDS210, manufactured by Kenis) or a pressure controller (MFCS-FLEX, manufactured by FLUIGENT) was connected to an ethylenetetrafluoroethylene (ETFE) tube using a silicone tube, and the ETFE tube was connected to the microcapillary of the puncture device using Fluidic Connections (IDEX Health & Science, connection terminals). After the connection, the puncture device was mounted on a micromanipulator (InjectMan N12, manufactured by Eppendorf) installed in a microscope. Pure water produced by Direct-Q UV (manufactured by MILLIPORE) was used as fluid flowing through the pump and the like.

(10) Puncture of alveolar organoids using puncture device

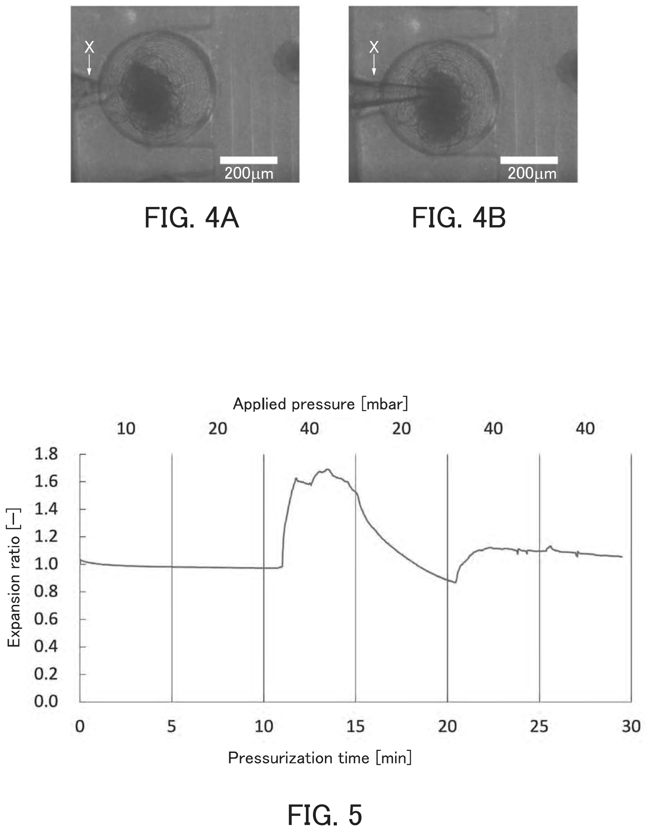

An alveolar organoid cultured in the above item (4) of Example 1 was punctured using the puncture device fabricated in the above item (8) of Example 1. A 35-mm dish containing the alveolar organoid cultured in the above item (4) of Example 1 was placed in the apparatus constructed in the above item (9) of Example 1. During the placement, care was taken to ensure that the puncture device could access the Matrigel in which the alveolar organoid was embedded and that the puncture device did not touch the dish wall. After the placement, the alveolar organoid on the dish was observed and punctured under a microscope. During the puncture, in order to operate the puncture device precisely and prevent damage to the puncture device or penetration into the alveolar organoid, the mode of the micromanipulator was switched to fine-tuning mode after the puncture device had reached the medium in the dish. During the puncture, the puncture device was moved to a position slightly above the alveolar organoid. After moving the puncture device as described above, the micromanipulator was operated so as to switch the moving direction of the Z-axis component to the axial direction, and further, the puncture device was moved downward along the Z-axis. After moving the puncture device as described above, as shown in FIG. 4A, the puncture device indicated by the arrow X in FIG. 4A was perpendicularly pressed against the alveolar organoid, thereby puncturing the alveolar organoid as shown in FIG. 4B.

(11) Measurement of expansion and contraction of alveolar organoid using puncture device

Next, expansion and contraction of an alveolar organoid were measured. Specifically, the alveolar organoid that had been punctured with the puncture device in the above item (10) of Example 1 was used. Fluid delivery and pressurization were performed with respect to the alveolar organoid using the syringe pump or the pressure controller of the apparatus constructed in the above item (9) of Example 1. The response of the alveolar organoid to the fluid delivery and the pressurization was recorded as a video using a camera (manufactured by Canon). After the video capturing, the time-dependent change in the area of the alveolar organoid in response to the fluid delivery and the pressurization was calculated using video analysis software (video editing and analysis software VW-H2MA, manufactured by KEYENCE). The conditions for the fluid delivery and the pressurization were as follows. The calculation was performed for one sac-like tissue at the punctured site of the alveolar organoid. In this calculation, the area of the alveolar organoid immediately after being punctured on an image was taken as 1, and the expansion and contraction of the alveolar organoid relative to this area was calculated (i.e., the expansion ratio). The results thereof are shown in FIG. 5.

(Conditions for fluid delivery and pressurization)

| Conditions for fluid delivery: | 50 $\mu$l/hours |
|---|---|

(continued)

| 0 to 5 minutes: | 10 mbar |
|---|---|
| 5 to 10 minutes: | 20 mbar |
| 10 to 15 minutes: | 40 mbar |
| 15 to 20 minutes: | 20 mbar |
| 20 to 30 minutes: | 40 mbar |

FIG. 5 is a graph showing the expansion ratio of the organoid. In FIG. 5, the horizontal axis indicates the pressurization time, and the vertical axis indicates the expansion ratio. As can be seen from FIG. 5, when a pressure of 40 mbar was applied to the alveolar organoid via the puncture device, the expansion ratio increased, that is, the alveolar organoid was expanded (10 to 15 minutes). Further, when the applied pressure was reduced, the expansion ratio of the alveolar organoid reduced, that is, the alveolar organoid was contracted (15 to 20 minutes). Still further, when the applied pressure was increased after the contraction of the alveolar organoid, the expansion ratio increased, that is, the alveolar organoid was expanded (20 to 30 minutes). These results demonstrate that the alveolar organoid provided with the puncture device allows access to the lumen of the alveolar organoid and that the alveolar organoid can be expanded and contracted by applying pressure or reduced pressure to the lumen of the alveolar organoid.

Example 2

Pulmonary acinus-like organoids were constructed, and they were evaluated using the device of the present disclosure.

(1) Construction of pulmonary acinus-like organoids

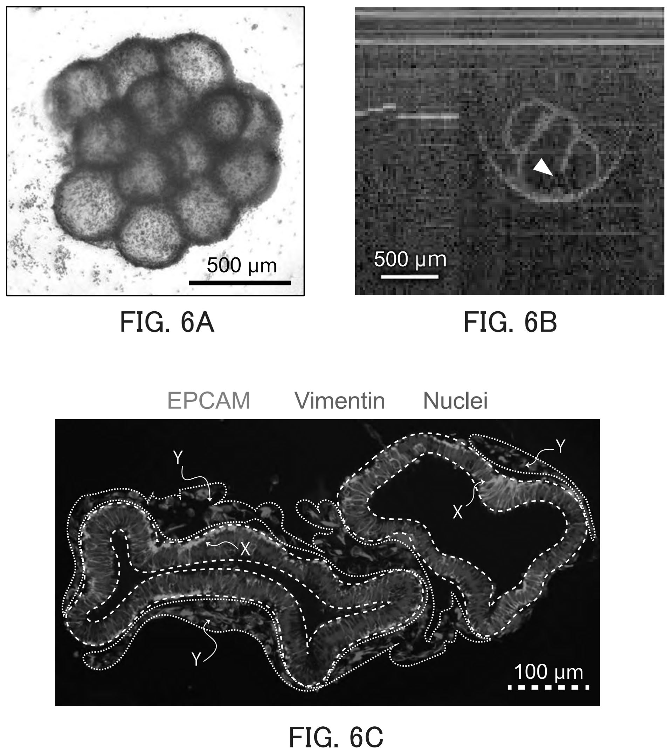

First, pulmonary acinus-like organoids were constructed using the spheroids obtained in the above item (3) of Example 1. The spheroids were collected from the wells into a 2 ml tube, and 500 $\mu$l of the maintenance medium was re-added to the wells after the collection. After the re-addition, the maintenance medium in which the spheroids were suspended was transferred to a 6-cm Petri dish (manufactured by Falcon, 351007) with a micropipette. Thereafter, the Petri dish was observed under a stereomicroscope (manufactured by Olympus Corporation, SZ61), and about 5 to 50 spheroids were collected using a micropipette. After the collection, the collected spheroids were seeded into one well of a Prime-Surface® 96 V plate (manufactured by Sumitomo Bakelite Co., Ltd., MS-9096V). After the seeding, 150 $\mu$l of differentiation medium was added to each well of the plate. The differentiation medium was prepared by adding, to the above-described maintenance medium, 1000-fold diluted SB431542 (10 mM, manufactured by FUJIFILM Wako Pure Chemical Corporation), 1000-fold diluted Recombinant Human Noggin (100 $\mu$g/ml, manufactured by R&D Systems, RSD-6057-NG-025-25), and 10-fold diluted Matrigel® Growth Factor Reduced (Matrigel, manufactured by Corning). After the addition, the spheroids were cultured in an incubator for 3 to 8 days. After the culture, the structures of the thus-constructed organoids were observed. The observation was performed using a microscope (BZ-9000, manufactured by KEYENCE CORPORATION) and an optical tomography system (IVS-2000-HR, manufactured by Sanyo Engineering & Construction Inc.). Frozen sections of the organoids were prepared in the same manner as described in the above item (6) of Example 1. The frozen sections were immunostained and observed using an optical microscope (BZ-9000, manufactured by KEYENCE CORPORATION). In the immunostaining, EPCAM antibody (manufactured by SantaCruz, Cat No.: #sc-66020, diluted 500-fold) and vimentin antibody (manufactured by Cell Signaling, Cat No.: #5741, diluted 500-fold) were used as primary antibodies, Goat anti-Mouse IgG (H+L) Cross-Adsorbed Secondary Antibody, Alexa Fluor™ 488 (manufactured by Invitrogen, Cat No.: #A-11001, diluted 500-fold) and Goat anti-Rabbit IgG (H+L) Cross-Adsorbed Secondary Antibody, Alexa Fluor™ 546 (manufactured by Invitrogen, Cat No.: #A-11010, diluted 500-fold) were used as secondary antibodies, and Hoechst 33342 (manufactured by Thermo Fisher Scientific, diluted 500-fold) was used for nuclear staining. The results thereof are shown in FIG. 6.

FIG. 6 shows photographs showing the results of observing a constructed organoid. FIG. 6A shows a bright-field photograph of the organoid, FIG. 6B shows an optical coherence tomography image of the organoid, and FIG. 6C shows a photograph of an immunostained image of the organoid. The scale bar in FIGs. 6A and 6B indicates 500 $\mu$m, and the scale bar in FIG. 6C indicates 100 $\mu$m. In FIG. 6B, the arrow indicates a portion where lumens communicate with each other. In FIG. 6C, portions enclosed by the dot-dashed line (arrow X) indicate the localization of EPCAM, and portions enclosed by the dashed line (arrow Y) indicate the localization of vimentin. As can be seen from FIG. 6A, the constructed organoid had a structure similar to a bunch of grapes. As can be seen from FIGs. 6B and 6C, communication between lumens was observed in the constructed organoid. Further, as can be seen from FIG. 6C, the localization of EPCAM was observed in the lumen-side cell layer, and the localization of vimentin was observed in the outer cell layer. These results confirm that the organoid like a pulmonary acinus (which is a cluster of alveoli connected to a single terminal respiratory bronchiole and an anatomical unit central to an understanding of pulmonary diseases).

(2) Measurement of expansion and contraction of alveolar organoids constituting pulmonary acinus-like organoid using puncture device

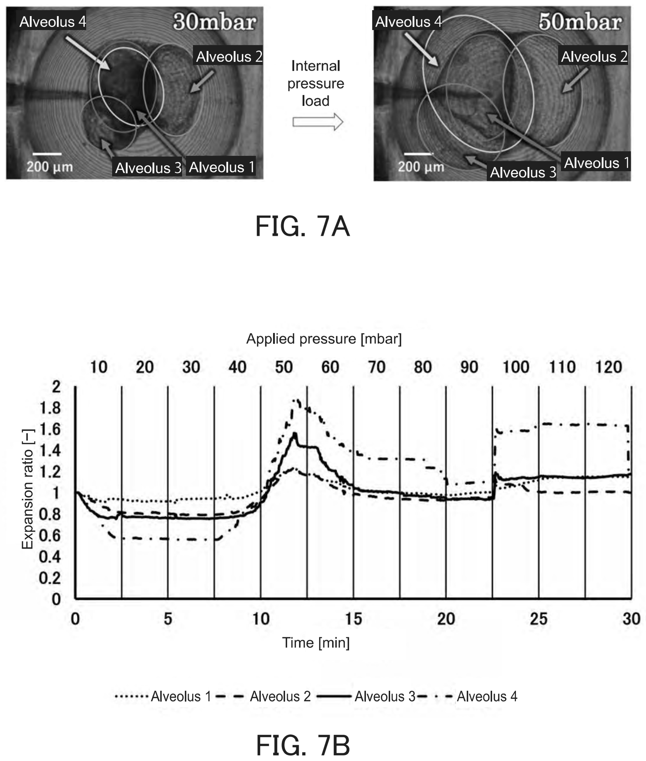

Next, expansion and contraction of alveolar organoids constituting a pulmonary acinus-like organoid were measured. Specifically, a pulmonary acinus-like organoid constructed in the above item (1) of Example 2 was punctured in the same manner as in the above item (10) of Example 1. Fluid delivery and pressurization were performed with respect to the lumen of the pulmonary acinus-like organoid using the syringe pump or the pressure controller of the apparatus constructed in the above item (9) of Example 1. The response of the alveoli to the fluid delivery and the pressurization was recorded as a video using a camera (manufactured by Canon). After the video capturing, the time-dependent change in the area of the alveoli in response to the fluid delivery and the pressurization was calculated using video analysis software (video editing and analysis software VW-H2MA, manufactured by KEYENCE). The conditions for the fluid delivery and the pressurization were as follows. The calculation was performed for four alveolar-like structures constituting the pulmonary acinus-like organoid. In this calculation, the area of the alveoli immediately after being punctured on an image was taken as 1, and the expansion and contraction of the alveoli relative to this area was calculated (i.e., the expansion ratio). The results thereof are shown in FIG. 7.

(Conditions for fluid delivery and pressurization)

| Conditions for fluid delivery: | 50 $\mu$l/hours |
|---|---|
| 0 to 2.5 minutes: | 10 mbar |
| 2.5 to 5 minutes: | 20 mbar |
| 5 to 7.5 minutes: | 30 mbar |
| 7.5 to 10 minutes: | 40 mbar |
| 10 to 12.5 minutes: | 50 mbar |
| 12.5 to 15 minutes: | 60 mbar |
| 15 to 17.5 minutes: | 70 mbar |
| 17.5 to 20 minutes: | 80 mbar |
| 20 to 22.5 minutes: | 90 mbar |
| 22.5 to 25 minutes: | 100 mbar |
| 25 to 27.5 minutes: | 110 mbar |
| 27.5 to 30 minutes: | 120 mbar |

FIG. 7 shows photographs and a graph, showing the results obtained when expansion and contraction of alveolar-like structures constituting a pulmonary acinus-like organoid were caused using the puncture device. FIG. 7A shows photographs showing the expansion of the lumens of the pulmonary acinus-like organoid, and FIG. 7B shows a graph showing the expansion ratio of each alveolar-like structure. In FIG. 7A, the scale bar indicates 200 $\mu$m, the photograph on the left is a photograph of the pulmonary acinus-like organoid when subjected to an internal pressure load of 30 mbar, and the photograph on the right is a photograph of the pulmonary acinus-like organoid when subjected to an internal pressure load of 50 mbar. In FIG. 7B, the vertical axis indicates the expansion ratio (relative value), and the horizontal axis indicates the time (minutes). As can be seen from FIG. 7A, it was found that, when the internal pressure load was increased from 30 mbar to 50 mbar, the lumens of the alveolar-like structures (alveoli 1 to 4) constituting the pulmonary acinus-like organoid were expanded. As can be seen from FIG. 7B, when the alveolar-like structures were pressurized to 50 mbar via the puncture device, the expansion ratio increased, that is, the alveolar-like structures were expanded (10 to 12.5 minutes). From these results, it was found that, even if the puncture device punctured only one site among the alveolar-like structures constituting the alveolar-like structure, the alveolar-like structures constituting the pulmonary acinus organoid were all expanded. In other words, it was found that the alveolar-like structures constituting the pulmonary acinus organoid were in communication with each other internally.

(3) Evaluation of phenotypes of pulmonary acinus-like organoids induced by adding drugs

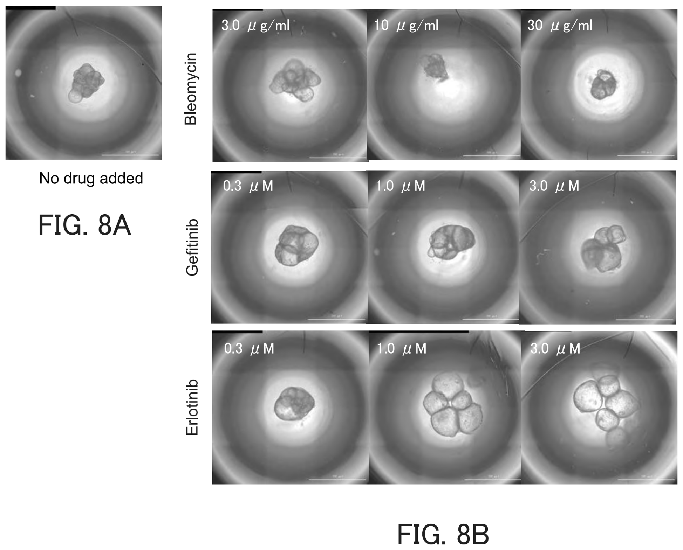

The organoids constructed in the above item (1) of Example 2 were evaluated for their phenotype induced by adding drugs. Specifically, the pulmonary acinus-like organoids constructed in the above item (1) of Example 2 were used, and on day 3 after the construction of the organoids, the culture medium was replaced with a drug-containing medium. The drug-containing medium was prepared by adding a drug with a high incidence rate of drug-induced interstitial pneumonia to the above-described maintenance medium. The drug used was bleomycin (10 mg/ml, manufactured by Nippon Kayaku Co., Ltd., 4987170006109), gefitinib (1 mM, manufactured by Tokyo Chemical Industry Co., Ltd., G0546), or erlotinib (1 mM, manufactured by Tokyo Chemical Industry Co., Ltd., E1404), each diluted 333-fold, 1000-fold diluted, or 3333-fold. After the replacement, the organoids were cultured in an incubator for 3 to 4 days. After the culture, the pulmonary acinus-like organoids were observed using a microscope (manufactured by BioTek, Cytation 5). The results thereof are shown in FIG. 8.

FIG. 8 shows photographs showing the results of adding various drugs to the pulmonary acinus-like organoids. In FIG. 8, the scale bar indicates 2000 $\mu$m. FIG. 8A shows a photograph of a pulmonary acinus-like organoid when no drug was added, and FIG. 8B shows photographs of pulmonary acinus-like organoids when the respective drugs were added. As can be seen from FIG. 8, when the bleomycin was added, the overall structure of the pulmonary acinus-like organoid exhibited a tendency toward contraction. Further, it was found, when the erlotinib was added, the size of the lumen of the pulmonary acinus-like organoid increased in a concentration-dependent manner.

(4) Evaluation of expansion and contraction of alveolar organoids treated by adding bleomycin using puncture device

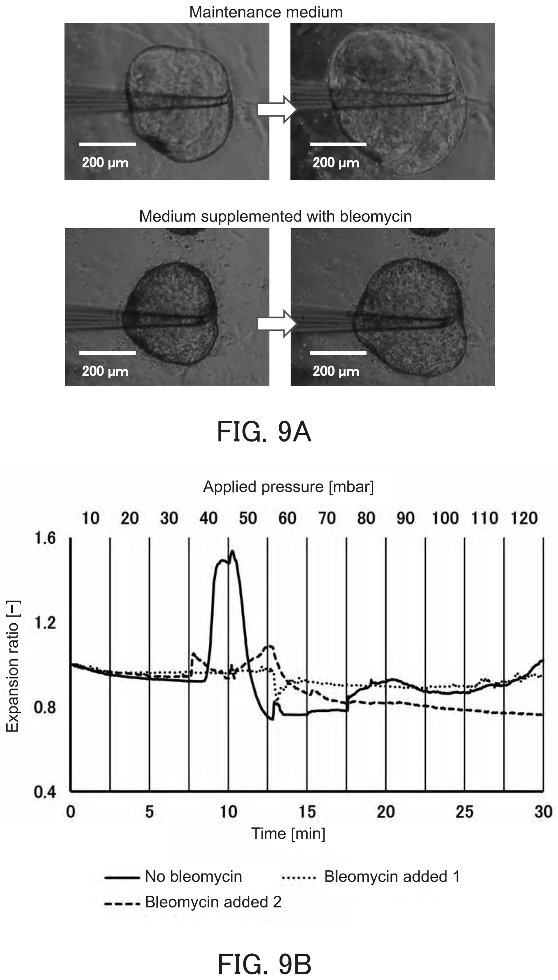

The expansion and contraction of alveolar organoids treated by adding bleomycin were examined using the puncture device. Specifically, the alveolar organoids cultured in the above item (4) of Example 1 were used, and on day 3 after the construction of the alveolar organoids, the culture medium was replaced with a medium supplemented with bleomycin. The medium supplemented with bleomycin was prepared by adding 333-fold diluted bleomycin (10 mg/ml, manufactured by Nippon Kayaku Co., Ltd., 4987170006109) to the above-described maintenance medium. After the replacement, the organoids were cultured in an incubator for 3 to 4 days. After the culture, the alveolar organoids were punctured in the same manner as in the above item (10) of Example 1. Fluid delivery and pressurization were performed with respect to the lumens of the alveolar organoids using the syringe pump or the pressure controller of the apparatus constructed in the above item (9) of Example 1. The response of each alveolar organoid to the fluid delivery and the pressurization was recorded as a video using a camera (manufactured by Canon). After the video capturing, the time-dependent change in the area of the alveolar organoid in response to the fluid delivery and the pressurization was calculated using video analysis software (video editing and analysis software VW-H2MA, manufactured by KEYENCE). The conditions for the fluid delivery and the pressurization were as follows. In this calculation, the area of the alveolar organoid immediately after being punctured on an image was taken as 1, and the expansion and contraction of the alveolar organoid relative to this area was calculated (i.e., the expansion ratio). A control experiment was performed in the same manner, except that the maintenance medium was used instead of the medium supplemented with bleomycin. The results thereof are shown in FIG. 9.

(Conditions for fluid delivery and pressurization)

| Conditions for fluid deli very: | 50 $\mu$l/hours |
| --- | --- |
| 0 to 2.5 minutes: | 10 mbar |
| 2.5 to 5 minutes: | 20 mbar |
| 5 to 7.5 minutes: | 30 mbar |
| 7.5 to 10 minutes: | 40 mbar |
| 10 to 12.5 minutes: | 50 mbar |
| 12.5 to 15 minutes: | 60 mbar |
| 15 to 17.5 minutes: | 70 mbar |
| 17.5 to 20 minutes: | 80 mbar |
| 20 to 22.5 minutes: | 90 mbar |
| 22.5 to 25 minutes: | 100 mbar |
| 25 to 27.5 minutes: | 110 mbar |

(continued)

| 27.5 to 30 minutes: | 120 mbar |

FIG. 9 shows photographs and a graph, showing the results obtained when expansion and contraction of an alveolar organoid treated by adding bleomycin were caused using the puncture device. In FIG. 9A, the upper row shows photographs showing the expansion of the lumen of the alveolar organoid in the control (the maintenance medium) and the lower row shows photographs showing the expansion of the lumen of the alveolar organoid treated by adding the bleomycin; and FIG. 9B is a graph showing the expansion ratio of the alveolar organoids. In FIG. 9A, the scale bar indicates 200 $\mu$m, the photographs on the left are photographs of the alveolar organoids when subjected to an internal pressure load of 10 mbar, and the photographs on the right are photographs of the alveolar organoids when subjected to an internal pressure load of 50 mbar (the maintenance medium) or 60 mbar (the medium supplemented with the bleomycin). In FIG. 9B, the vertical axis indicates the expansion ratio (relative value), and the horizontal axis indicates the time (minutes). As can be seen from FIG. 9A, it was found that, when the internal pressure load was increased, expansion of the lumen of the alveolar organoid was observed in the control. On the other hand, in the case where bleomycin was added, the expansion of the lumen of the alveolar organoid lumen was not observed, in contrast to the case of the control. As can be seen from FIG. 9B, in the control, the expansion ratio of the alveolar organoid increased as a result of pressurization to 40 to 50 mbar via the puncture device. On the other hand, in the case where the bleomycin was added, an increase in the expansion ratio of the alveolar organoid was not observed at any applied pressure. These results suggest that bleomycin induces fibrosis in organoids, thereby causing the organoids to resist pressure loads generated by luminal perfusion and making the lumens less expandable.

While the present invention has been described above with reference to exemplary embodiments and examples, the present disclosure is by no means limited thereto. Various changes and modifications that may become apparent to those skilled in the art may be made in the configuration and specifics of the present invention without departing from the scope of the present disclosure.

This application is based upon and claims the benefit of priority from Japanese Patent Application No. 2023-069093 filed on April 20, 2023, the disclosure of which is incorporated herein its entirety by reference.

Patents, patent applications, and references cited in the present specification are incorporated herein in their entirety by reference, as if fully and specifically set forth herein.

<Supplementary Notes>

Part or the whole of the exemplary embodiments and examples disclosed above can be described as in the following supplementary notes. It is to be noted, however, that the present invention is by no means limited thereto.

<Structure>

(Supplementary Note 1)

A structure including:

a sac-like cellular structure; and
a tubular body,
wherein the cellular structure includes a lumen and a cell layer at least partially covering the lumen,
the cell layer is composed of cells, and
the tubular body is configured to allow the lumen of the cellular structure to be in communication with an outside of the cellular structure.

(Supplementary Note 2)

The structure according to Supplementary Note 1, wherein the cells include epithelial cells.

(Supplementary Note 3)

The structure according to Supplementary Note 2, wherein the epithelial cells include alveolar epithelial type II cells.

(Supplementary Note 4)

The structure according to any one of Supplementary Notes 1 to 3, wherein the cellular structure has apical polarity on its lumen side.

(Supplementary Note 5)

The structure according to any one of Supplementary Notes 1 to 4, wherein the cellular structure expresses a Zonula occludens (ZO)-1 gene and/or Crumbs (CRB) 3 on the lumen side.

(Supplementary Note 6)

The structure according to any one of Supplementary Notes 1 to 5, wherein the lumen contains a pulmonary surfactant protein, mucin, Clara cell secretion, and/or a lipid as its content.

(Supplementary Note 7)

The structure according to any one of Supplementary Notes 1 to 6, wherein the cellular structure is composed of a single layer of cells.

(Supplementary Note 8)

The structure according to any one of Supplementary Notes 1 to 7, wherein the tubular body has a diameter of 10 to 500 $\mu$m.

(Supplementary Note 9)

The structure according to any one of Supplementary Notes 1 to 8, wherein the cellular structure includes an organoid.

(Supplementary Note 10)

The structure according to Supplementary Note 9, wherein the cellular structure includes at least one organoid selected from the group consisting of pulmonary acinus organoids, alveolar organoids, airway organoids, intestinal organoids, small intestinal organoids, brain organoids, cortical organoids, retinal organoids, cystic organoids, gastric organoids, kidney organoids, bile duct organoids, esophageal organoids, and large intestinal organoids.

(Supplementary Note 11)

The structure according to any one of Supplementary Notes 1 to 10, wherein the cellular structure can be expanded and/or contracted by changing pressure applied to the lumen via the tubular body.

<Evaluation method>

(Supplementary Note 12)

An evaluation method using the structure according to any one of Supplementary Notes 1 to 11, including the steps of:

preparing a coexistence system in which a test substance and the cellular structure are present together; and
evaluating the cellular structure and/or the test substance present in the coexistence system.

(Supplementary Note 13)

The evaluation method according to Supplementary Note 12, wherein the cellular structure is evaluated by evaluating at least one selected from the group consisting of a shape, hardness, ratio of contraction or expansion, cell differentiation marker, fibrotic marker, aging marker, oxidative stress marker, and apoptosis marker of the cellular structure.

(Supplementary Note 14)

The evaluation method according to Supplementary Note 12 or 13, wherein the test substance is evaluated by evaluating dynamics of the test substance.

(Supplementary Note 15)

The evaluation method according to any one of Supplementary Notes 12 to 14, wherein the evaluation of the dynamics of the test substance is evaluation of at least one selected from the group consisting of a concentration of the test substance, a concentration of a metabolite of the test substance, and a partial pressure of a gas.

<Pulmonary Acinus Organoid>

(Supplementary Note 16)

A pulmonary acinus organoid including two or more sac-like cellular structures,

wherein each of the cellular structures includes a lumen and two cell layers at least partially covering the lumen,
each of the two cell layers includes alveolar epithelial cells, fibroblasts, and/or vascular endothelial cells, and
the lumen of one of the cellular structures is in communication with the lumen of the at least one other cellular structure.

(Supplementary Note 17)

The pulmonary acinus organoid according to claim 16, wherein

the cell layers include a lumen-side cell layer and a cell layer laminated on part or the whole of an outer peripheral surface of the lumen-side cell layer,
the lumen-side cell layer is composed of alveolar epithelial cells, and
the cell layer laminated on the lumen-side cell layer is composed of fibroblasts and/or vascular endothelial cells.

(Supplementary Note 18)

The pulmonary acinus organoid according to Supplementary Note 16 or 17, wherein the alveolar epithelial cells are alveolar epithelial type I cells, alveolar epithelial type II cells, and/or bronchiolar epithelial cells.

(Supplementary Note 19)

The pulmonary acinus organoid according to Supplementary Note 18, wherein the alveolar epithelial cells are alveolar epithelial type I cells.

(Supplementary Note 20)

The pulmonary acinus organoid according to Supplementary Note 19, wherein the alveolar epithelial type II cells are positive for SFTPA, SFTPB, SFTPC, SFTPD, EpCAM, ABCA3, DCLAMP, CEACAM6, SLC34A2, and/or LPCAT1.

(Supplementary Note 21)

The pulmonary acinus organoid according to any one of Supplementary Notes 16 to 20, wherein the fibroblasts are lung fibroblasts.

(Supplementary Note 22)

The pulmonary acinus organoid according to any one of Supplementary Notes 16 to 21, wherein the fibroblasts are positive for vimentin, ACTA2, COL1A1, ELN, FN, PDGFRA, POSTN, and/or TAGLN.

(Supplementary Note 23)

The pulmonary acinus organoid according to any one of Supplementary Notes 16 to 22, wherein each of the cellular structures has apical polarity on its lumen side.

(Supplementary Note 24)

The pulmonary acinus organoid according to any one of Supplementary Notes 16 to 23, wherein each of the cellular structures is an alveolar-like structure.

<Method for Producing Pulmonary Acinus Organoid>

(Supplementary Note 25)

A method for producing a pulmonary acinus organoid, including the step of culturing two or more spheroids in the presence of a differentiation-inducing factor for inducing differentiation into pulmonary acinus organoids,
wherein each of the spheroids is composed of lung progenitor cells, lung fibroblasts, and/or vascular endothelial cells.

(Supplementary Note 26)

The production method according to Supplementary Note 25, wherein each of the spheroids includes lung progenitor cells and lung fibroblasts.

(Supplementary Note 27)

The production method according to Supplementary Note 25 or 26, wherein the lung progenitor cells are positive for carboxypeptidase M (CPM), NK2 homeobox 1 (NKX2.1), SRY-box 9 (SOX) 9, SRY-box 2 (SOX2), and/or forkhead box protein 2 A (FOXA2).

(Supplementary Note 28)

The production method according to any one of Supplementary Note 25 to 27, wherein the lung progenitor cells are positive for CPM and/or NKX2.1.

(Supplementary Note 29)

The production method according to any one of Supplementary Notes 25 to 28, wherein the differentiation-inducing factor is a TGF-β1 inhibitor, a BMP inhibitor, a steroid agent, a phosphodiesterase inhibitor, KGF, a Wnt activator, a GSK3β inhibitor, a ROCK inhibitor, a MAP kinase inhibitor, EGF, and/or FGF.

(Supplementary Note 30)

The production method according to Supplementary Note 29, wherein the differentiation-inducing factor includes the TGF-β1 inhibitor and the BMP inhibitor.

(Supplementary Note 31)

The production method according to Supplementary Note 29 or 30, wherein the concentration of the TGF-β1 inhibitor is 100 nM to 100 $\mu$M.

(Supplementary Note 32)

The production method according to any one of Supplementary Notes 29 to 31, wherein the concentration of the BMP inhibitor is 100 pg/ml to 100 $\mu$g/ml.

(Supplementary Note 33)

The production method according to any one of Supplementary Notes 29 to 32, wherein the concentration of the steroid agent is 1 nM to 1 $\mu$M.

(Supplementary Note 34)

The production method according to any one of Supplementary Notes 29 to 33, wherein the concentration of the phosphodiesterase inhibitor is 1 nM to 1 $\mu$M.

(Supplementary Note 35)

The production method according to any one of Supplementary Notes 29 to 34, wherein the concentration of the KGF is 10 ng/ml to 1 $\mu$g/ml.

(Supplementary Note 36)

The production method according to any one of Supplementary Notes 29 to 35, wherein the concentration of the Wnt activator is 1 nM to 100 μM.

(Supplementary Note 37)

The production method according to any one of Supplementary Notes 29 to 36, wherein the concentration of the GSK3β inhibitor is 1 nM to 100 μM.

(Supplementary Note 38)

The production method according to any one of Supplementary Notes 29 to 37, wherein the concentration of the ROCK inhibitor is 1 nM to 50 μM.

(Supplementary Note 39)

The production method according to any one of Supplementary Notes 29 to 38, wherein the concentration of the MAP kinase inhibitor is 1 nM to 100 μM.

(Supplementary Note 40)

The production method according to any one of Supplementary Notes 29 to 39, wherein the concentration of the EGF is 1 ng/ml to 1 μg/ml.

(Supplementary Note 41)

The production method according to any one of Supplementary Notes 29 to 40, wherein the concentration of the FGF is 1 ng/ml to 1 μg/ml.

INDUSTRIAL APPLICABILITY

As specifically described above, the present disclosure can provide a structure that allows access to the lumen of a cellular structure. Therefore, it can be said that the structure of the present disclosure is very useful in the field of drug discovery.

[Sequence Listing]

P23028WO.xml

**Claims**

1. A structure comprising:

    a sac-like cellular structure; and
    a tubular body,
    wherein the cellular structure comprises a lumen and a cell layer at least partially covering the lumen,

    the cell layer is composed of cells, and
    the tubular body is configured to allow the lumen of the cellular structure to be in communication with an outside of the cellular structure.

2. The structure according to claim 1, wherein the cells comprise epithelial cells.

3. The structure according to claim 2, wherein the epithelial cells comprise alveolar epithelial type II cells.

4. The structure according to any one of claims 1 to 3, wherein the cellular structure has apical polarity on its lumen side.

5. The structure according to any one of claims 1 to 3, wherein the cellular structure expresses a Zonula occludens (ZO)-1 gene and/or Crumbs (CRB) 3 on the lumen side.

6. The structure according to any one of claims 1 to 3, wherein the lumen contains a pulmonary surfactant protein, mucin, Clara cell secretion, and/or a lipid as its content.

7. The structure according to any one of claims 1 to 3, wherein the cellular structure is composed of a single layer of cells.

8. The structure according to any one of claims 1 to 3, wherein the tubular body has a diameter of 10 to 500 μm.

9. The structure according to any one of claims 1 to 3, wherein the cellular structure comprises an organoid.

10. The structure according to claim 9, wherein the cellular structure comprises at least one organoid selected from the group consisting of pulmonary acinus organoids, alveolar organoids, airway organoids, intestinal organoids, small intestinal organoids, brain organoids, cortical organoids, retinal organoids, cystic organoids, gastric organoids, kidney organoids, bile duct organoids, esophageal organoids, and large intestinal organoids.

11. The structure according to any one of claims 1 to 3, wherein the cellular structure can be expanded and/or contracted by changing pressure applied to the lumen via the tubular body.

12. An evaluation method using the structure according to claim 1, comprising the steps of:

    preparing a coexistence system in which a test substance and the cellular structure are present together; and
evaluating the cellular structure and/or the test substance present in the coexistence system.

13. The evaluation method according to claim 12, wherein the cellular structure is evaluated by evaluating at least one selected from the group consisting of a shape, hardness, ratio of contraction or expansion, cell differentiation marker, fibrotic marker, aging marker, oxidative stress marker, and apoptosis marker of the cellular structure.

14. The evaluation method according to claim 12 or 13, wherein the test substance is evaluated by evaluating dynamics of the test substance.

15. The evaluation method according to claim 12 or 13, wherein the evaluation of the dynamics of the test substance is evaluation of at least one selected from the group consisting of a concentration of the test substance, a concentration of a metabolite of the test substance, and a partial pressure of a gas.

16. A pulmonary acinus organoid comprising two or more sac-like cellular structures,

wherein each of the cellular structures comprises a lumen and two cell layers at least partially covering the lumen,
each of the two cell layers comprises alveolar epithelial cells, fibroblasts, and/or vascular endothelial cells, and
the lumen of one of the cellular structures is in communication with the lumen of the at least one other cellular structure.

17. The pulmonary acinus organoid according to claim 16, wherein

the cell layers comprise a lumen-side cell layer and a cell layer laminated on part or the whole of an outer peripheral surface of the lumen-side cell layer,
the lumen-side cell layer is composed of alveolar epithelial cells, and
the cell layer laminated on the lumen-side cell layer is composed of fibroblasts and/or vascular endothelial cells.

18. The pulmonary acinus organoid according to claim 16 or 17, wherein the alveolar epithelial cells are alveolar epithelial type I cells, alveolar epithelial type II cells, and/or respiratory bronchiolar epithelial cells.

19. The pulmonary acinus organoid according to claim 18, wherein the alveolar epithelial cells are alveolar epithelial type II cells.

20. The pulmonary acinus organoid according to claim 19, wherein the alveolar epithelial type II cells are positive for SFTPA, SFTPB, SFTPC, SFTPD, Ep-CAM, ABCA3, DCLAMP, CEACAM6, SLC34A2, and/or LPCAT1.

21. The pulmonary acinus organoid according to any one of claims 16 to 20, wherein the fibroblasts are lung fibroblasts.

22. The pulmonary acinus organoid according to any one of claims 16 to 21, wherein the fibroblasts are positive for vimentin, ACTA2, COL1A1, ELN, FN, PDGFRA, POSTN, and/or TAGLN.

23. The pulmonary acinus organoid according to any one of claims 16 to 22, wherein each of the cellular structures has apical polarity on its lumen side.

24. The pulmonary acinus organoid according to any one of claim 16 to 23, wherein each of the cellular structures is an alveolar-like structure.

25. A method for producing a pulmonary acinus organoid, comprising the step of culturing two or more spheroids in the presence of a differentiation-inducing factor for inducing differentiation into pulmonary acinus organoids,
wherein each of the spheroids is composed of lung progenitor cells, lung fibroblasts, and/or vascular endothelial cells.

26. The production method according to claim 25, wherein each of the spheroids comprises lung progenitor cells and lung fibroblasts.

27. The production method according to claim 25 or 26, wherein the lung progenitor cells are positive for carboxypeptidase M (CPM), NK2 homeobox 1 (NKX2.1), SRY-box 9 (SOX) 9, SRY-box 2 (SOX2), and/or forkhead box protein 2 A (FOXA2).

28. The production method according to any one of claim 25 to 27, wherein the lung progenitor cells are positive for CPM and/or NKX2.1.

29. The production method according to any one of claims 25 to 28, wherein the differentiation-inducing factor is a TGF-β1 inhibitor, a BMP inhibitor, a steroid agent, a phosphodiesterase inhibitor, KGF, a Wnt activator, a GSK3β inhibitor, a ROCK inhibitor, a MAP kinase inhibitor, EGF, and/or FGF.

30. The production method according to claim 29, wherein the differentiation-inducing factor comprises the TGF-β1 inhibitor and the BMP inhibitor.

31. The production method according to claim 29 or 30, wherein the concentration of the TGF-β1 inhibitor is 100 nM to 100 μM.

32. The production method according to any one of claims 29 to 31, wherein the concentration of the BMP inhibitor is 100 pg/ml to 100 μg/ml.

Average value of major and minor axes [μm]

FIG. 1A

Circularity [−]

FIG. 1B

Average value of major and minor axes [μm]

FIG. 1C

Circularity [−]

FIG. 1D

FIG. 2

FIG. 3

FIG. 4A

FIG. 4B

FIG. 5

FIG. 6A

FIG. 6B

FIG. 6C

FIG. 7A

FIG. 7B

No drug added

FIG. 8A

FIG. 8B

Maintenance medium

Medium supplemented with bleomycin

## FIG. 9A

FIG. 9B

## INTERNATIONAL SEARCH REPORT

| International application No. |
|---|
| **PCT/JP2024/015630** |

| A. | CLASSIFICATION OF SUBJECT MATTER |
|---|---|

*C12N 5/071*(2010.01)i; *C12Q 1/02*(2006.01)i
FI: C12N5/071; C12Q1/02

According to International Patent Classification (IPC) or to both national classification and IPC

| B. | FIELDS SEARCHED |
|---|---|

Minimum documentation searched (classification system followed by classification symbols)

C12N5/071; C12Q1/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2019/087988 A1 (PUBLIC UNIVERSITY CORPORATION YOKOHAMA CITY UNIVERSITY) 09 May 2019 (2019-05-09) paragraphs [0009], [0016]-[0037], fig. 1-3 | 1, 2, 9-15 |
| A | | 3-8, 16-32 |
| X | JP 2023-035224 A (SHISEIDO COMPANY, LTD.) 13 March 2023 (2023-03-13) paragraphs [0010]-[0029], fig. 1, 2 | 1, 2, 9-15 |
| A | | 3-8, 16-32 |
| A | WO 2023/286852 A1 (CELLFIBER CO., LTD.) 19 January 2023 (2023-01-19) entire text, all drawings | 1-32 |

☐ Further documents are listed in the continuation of Box C.　☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **02 July 2024** | **16 July 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

International application No.

**PCT/JP2024/015630**

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  [✓] forming part of the international application as filed.

    b.  [ ] furnished subsequent to the international filing date for the purposes of international search (Rule 13*ter*.1(a)),

        [ ] accompanied by a statement to the effect that the sequence listing does not go beyond the disclosure in the international application as filed.

2.  [ ] With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

INTERNATIONAL SEARCH REPORT
Information on patent family members

International application No.

**PCT/JP2024/015630**

| Patent document cited in search report | | | Publication date (day/month/year) | Patent family member(s) | | | Publication date (day/month/year) |
|---|---|---|---|---|---|---|---|
| WO | 2019/087988 | A1 | 09 May 2019 | US | 2020/0261619 | A1 | |
| | | | | paragraphs [0013], [0040]-[0065], Fig. 1-3 | | | |
| | | | | EP | 3705568 | A1 | |
| | | | | CN | 111386335 | A | |
| JP | 2023-035224 | A | 13 March 2023 | (Family: none) | | | |
| WO | 2023/286852 | A1 | 19 January 2023 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11299712 B

- JP 2023069093 A

**Non-patent literature cited in the description**

- **YAMAMOTO Y et al.** *Nat Methods*, November 2017, vol. 14 (11), 1097-1106
- **YAMAMOTO Y et al.** *Nat Methods*, 02 October 2017, vol. 14 (11), 1097-1106
- **KONISHI S et al.** *Stem Cell Reports.*, 24 December 2015, vol. 6 (1), 18-25
- **SPENCE JR et al.** Directed differentiation of human pluripotent stem cells into intestinal tissue in vitro. *Nature*, 2011, vol. 470, 105-9
- **SPENCE JR et al.** *Nature*, 12 December 2010, vol. 470 (7332), 105-9
- **WATSON CL et al.** An in vivo model of human small intestine using pluripotent stem cells. *Nat Med.*, 2014, vol. 20, 1310-4
- **LANCASTER MA et al.** Cerebral organoids model human brain development and microcephaly. *Nature*, 2013, vol. 501, 373-9
- **CAMP JG et al.** Human cerebral organoids recapitulate gene expression programs of fetal neocortex development. *Proc Natl Acad Sci U S A.*, 2015, vol. 112, 15672-7
- **MARIANI J et al.** FOXG1-Dependent Dysregulation of GABA/Glutamate Neuron Differentiation in Autism Spectrum Disorders. *Cell*, 2015, vol. 162, 375-390
- **CUGOLA FR et al.** The Brazilian Zika virus strain causes birth defects in experimental models.. *Nature*, 2016, vol. 534, 267-71
- **GARCEZ PP et al.** Zika virus impairs growth in human neurospheres and brain organoids. *Science*, 2016, vol. 352, 816-8

- **GABRIEL E et al.** CPAP promotes timely cilium disassembly to maintain neural progenitor pool. *EMBO J.*, 2016, vol. 35, 803-19
- **OTANI T et al.** 2D and 3D Stem Cell Models of Primate Cortical Development Identify Species-Specific Differences in Progenitor Behavior Contributing to Brain Size. *Cell Stem Cell*, 2016, vol. 18, 467-80
- **TUCKER BA et al.** Duplication of TBK1 Stimulates Autophagy in iPSC-derived Retinal Cells from a Patient with Normal Tension Glaucoma. *J Stem Cell Res Ther*, 2014, vol. 3, 161
- **SAMPAZIOTIS F et al.** Cholangiocytes derived from human induced pluripotent stem cells for disease modeling and drug validation. *Nat Biotechnol.*, 2015, vol. 33, 845-852
- **OGAWA M et al.** Directed differentiation of cholangiocytes from human pluripotent stem cells. *Nat Biotechnol.*, 2015, vol. 33, 853-61
- **TAKASATO M et al.** Kidney organoids from human iPS cells contain multiple lineages and model human nephrogenesis. *Nature*, 2015, vol. 526, 564-8
- **MCCRACKEN KW. et al.** *Nature*, 29 October 2014, vol. 516 (7531), 400-4
- **MUNERA JO et al.** *Cell Stem Cell*, 22 June 2017, vol. 21 (1), 51-64
- **CO, J.Y** ; **MARGALEF-CATALA, M** ; **MONACK, D.M. et al.** Controlling the polarity of human gastrointestinal organoids to investigate epithelial biology and infectious diseases. *Nat Protoc*, 2021, vol. 16, 5171-5192